# EUROPEAN PATENT APPLICATION

(11) **EP 2 940 015 A1**
(43) Date of publication of application: **04.11.2015**
(21) Application number: 13866653.2
(22) Date of filing: 31.10.2013
(51) Int. Cl.: C07D 403/10, C08G 73/14, C07F 1/00

(54) **NOVEL POLYAMIDEIMIDE HAVING LOW THERMAL EXPANSION COEFFICIENT**

(30) Priority: 27.12.2012 KR 20120155221
(71) Applicant: Korea Advanced Institute of Science and Technology, Daejeon 305-701 (KR)
(72) Inventor: KIM, Sang Youl, Daejeon 305-701 (KR); KIM, Sun Dal, Daejeon 305-701 (KR)
(74) Representative: Epping - Hermann - Fischer
(86) International application number: PCT/KR2013/009763
(87) International publication number: WO 2014/104557

(57) **Abstract**

The present invention relates to a polyamideimide comprising an asymmetric dicarboxylic acid derivative wherein, from among a cyclic group A and a cyclic group B, two substitutents groups R and R' are bonded only to the cyclic group A on one side. A polymer produced by means of the present invention can have adequate solubility with respect to organic solvents, high thermal stability, a high glass transition temperature, high transparency and a low thermal expansion coefficient, and is able not only to satisfy requirements for core substrate materials of flexible displays but also to be used as a material for various soft electronic data devices.

## Description

### Field of Invention

The present invention relates to a soluble aromatic polyamideimide having asymmetric diamine monomer with two substituents attached to the aromatic ring of said amine and the method thereof. More specifically, the present invention relates to a soluble aromatic polyamideimide with high transparency and a low thermal expansion coefficient, a high glass transition temperature and high thermal stability, and adequate solubility with respect to organic solvents; and the method thereof.

### Background

Glass substrate used in the conventional flat display has advantages such as high heat resistance, transparency, barrier property. However, when dropped, the glass substrate can be easily broken with no flexibility and its heavy property with respect to the thinness enforces research to replace glass substrate to realize flexible display.

Moreover to apply the TFT manufacturing process controlling electrical signal of pixel to plastic display, the plastic substrate should endure high temperature(Tg, Td > 350°C)
with low thermal expansion coefficient(below 20 ppm / °C), satisfy the requirement such as chemical resistance, low permeability of oxygen and moisture, transparency above 85 % as well as low manufacturing cost. Currently, the available candidate to satisfy the said requirement is polyimide as plastic substrate.

The said polyimide(PI) is polymer with advantage such as relatively low crystallinity or amorphous structure, easy manufacturing process, easy process to make film, no crosslinkable moieties necessary for curing. Also polyimide does have polymeric properties such as high transparency, excellent flame and chemical resistance, excellent mechanical and electrical properties due to the rigid chain as well as dimensional stability, using PI as electrical and electronical material in the field of car, aerospace, flexible circuit board, liquid crystal alignment film for LCD, adhesive, as well as coating material.

However, even though polyimide is high performance polymer with excellent thermal stability, mechanical properties, chemical resistance and electrical properties, it does not satisfy the requirement of basic criteria of display area such as colorless transparency, and the thermal coefficient should be further lowered. For example, KAPTON sold by Dupont has thermal coefficient of about 30 ppm/°C, below the criteria for the plastic substrate for display. Therefore, research for minimizing change in thermal history and optical properties while maintaining the basic properties of polyimide is underway.

In general, aromatic polyimide is dark brown color due to the charge transfer complex(CT-complex) induced by π electrons of benzene within main chain of polyimide.

In general, the wavelength below 400 nm to 500 nm of visible light is absorbed by polyimide, and therefore significant coloration of polyimides occurs. To lower CT-complex formation of aromatic polyimide, introducing electron-withdrawing functional group such as trifluoromethyl(-CF3), sulfon(-SO2) to the main chain of polyimide is necessary to limit the movement of π electron. Also introducing alicyclic structure to main chain of polyimide instead of benzene can reduce the coloration of polyimide film.

Meanwhile, polyamideimide's properties such as thermal resistance, mechanical strength, electrical properties are excellent, and therefore polyamideimide has been widely used as industrial materials in the electrical, mechanical, electronic and aerospace fields. Also, polyamideimide's structure is different from that of polyimide and is known to be soluble in organic solvent, allowing for the application for enamel varnish, coating for electrical insulation.

Meanwhile, as related art to enhance the properties of the said polyamideimide, JP 2010-106225(2010.05.13.) teaches polyimide with trifluoromethyl and amide, and the manufacturing method thereof, and JP 2012-77144(2012.04.19.) teaches polyamideimide with trifluoromethyl, and the manufacturing method thereof for the usage in the flexible display as substrate.

However, despite the numerous commercialized polymers for the display field including related art, it is still necessary to further conduct research on the polyamideimide for the flexible display with low thermal expansion coefficient, high solubility, transparency as well as thermal stability.

### Detailed Description

### Technical Goal

The first goal of the present invention is to provide novel dicarboxylic acid derivatives as monomer of polyamideimide and the preparing method thereof, which can be applied to the plastic substrate in the flexible display.

Also the second goal of the present invention is to provide novel polyamideimide and the preparing method thereof with low thermal expansion coefficient, which can be applied to the plastic substrate in the flexible display by polymerization of the said novel dicarboxylic acid derivatives with known diamine, or polymerization of the known dicaboxylic acid derivaves with novel diamine monomer.

Also, the third goal of the present invention is novel diamine monomer with two substitutents resulting in the asymmetrical diamine and the preparation method thereof for the preparation of said novel polyamideimide.

### Problem Solving Action.

The present invention provides with dicarboxylic acid derivatives with diamine compound, using novel asymmetrical diamine which is unsymmetrically substituted with two substituents R and R' on the one side of the ring of diamine compound, and the preparation method thereof; polyamideimide containing said dicarboxylic acid derivatives and the preparation method thereof; to overcome the disadvantage of known polyimide.

In detail, the present invention provides with asymmetrical dicarboxylic acid derivatives represented by Formula A, its alkali metal salt or alkaline earth metal salt, and the preparation method thereof, wherein said dicarboxylic acid derivatives is substituted with two substituents R and R', asymmetrically attached to one side of cyclic group A of cyclic group A and cyclic group B in diamine compounds:

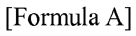

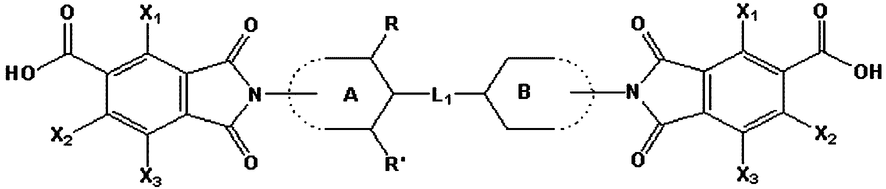

In formula A,
wherein R, R', X1, X2 and X3 are identical to or different from each other, and are independently selected from the group consisting of C1 to C12 alkyl group unsubstituted or substituted with one or more halogen; C1 to C12 alkoxy group unsubstituted or substituted with one or more halogen; C2 to C12 alkenyl group unsubstituted or substituted with one or more halogen; C2 to C12 alkynyl group unsubstituted or substituted with one or more halogen; C4 to C30 cycloalkyl group unsubstituted or substituted with one or more halogen; C4 to C30 cycloalkenyl group unsubstituted or substituted with one or more halogen; C6 to C30 aryl group unsubstituted or substituted with one or more halogen, C1 to C12 alkyl group, C1 to C12 alkoxy group, C1 to C12 halogenated alkyl group and/or C1 to C12 halogenated alkoxy group; C3 to C30 heteroaryl group unsubstituted or substituted with one or more halogen, C1 to C12 alkyl group, C1 to C12 alkoxy group, C1 to C12 halogenated alkyl group and/or C1 to C12 halogenated alkoxy group; and C6 to C30 arylalkyl group unsubstituted or substituted with one or more halogen, C1 to C12 alkyl group, C1 to C12 alkoxy group, C1 to C12 halogenated alkyl group and/or C1 to C12 halogenated alkoxy group;
L1 represents a direct bond, -O-, -S-, -C(=O)O-, -OC(=O)-, -C(=O)-, -SO2-,-C(R1)(R2)-, -NR3, [21] where R1, R2 and R3 are identical to or different from each other, and are independently selected from the group consisting of hydrogen; C1 to C12 alkyl group unsubstituted or substituted with one or more halogen; C4 to C20 cycloalkyl group unsubstituted or substituted with one or more halogen; C6 to C20 aryl group unsubstituted or substituted with one or more halogen; and C3 to C20 heteroaryl group unsubstituted or substituted with one or more halogen;
said cyclic group 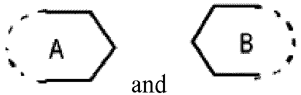 each independently represent C5 to C30 arylene or cycloalkylene unsubstituted or substituted with 5-membered or 6-membered ring which is unsubstituted or substituted or more halogen; or C5 to C30 heteroarylene or heterocycloalkylene unsubstituted or substituted with 5-membered or 6-membered ring which is unsubstituted or substituted or more halogen.

Also, the present invention provides with the novel polyamidemide derived from polymerization of dicarboxylic acid derivatives represented by said formula A and known diamine, and the preparation method thereof.

Also, the present invention provides with novel diamine represented by formula D and the preparation thereof, for the preparation of said dicarboxylic acid derivatives represented as formula A.

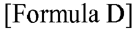

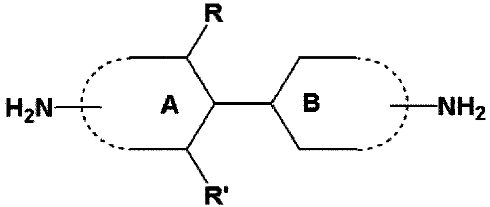

Wherein R, R' and cyclic group 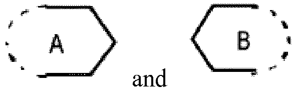 in formula D are the same as defined as above.

### Effect of the invention

In one embodiment, novel polyamideimide with amide and imide bond within main chain of polymer for the plastic substrate in the flexible display is synthesized by polymerization of dicarboxylic acid derivatives with known diamine.

The said polyamideimide of the present invention has bulky trifluoromethyl substituted on the main chain of polymer, resulting in the high solubility and transparency without lowering thermal resistance and high glass transient temperature due to rigidity of main polymer chain and the hydrogen bond between amides.

Also, the said polyamideimide has low thermal expansion coefficient by minimizing the changes in the structure of main chain of polymer by thermal source. Additionally, the fluorine in the main chain of polymer has low polarization, enabling the low moisture absorption, dielectrical constant and refractive index.

Therefore, the polyamideimide of the present invention can be maintained with excellent properties such as thermal, mechanical, and optical properties when it is made into film, therefore, allowing it usable as substrate material in the flexible display.

### BRIEF DESCRIPTION OF THE DRAWINGS

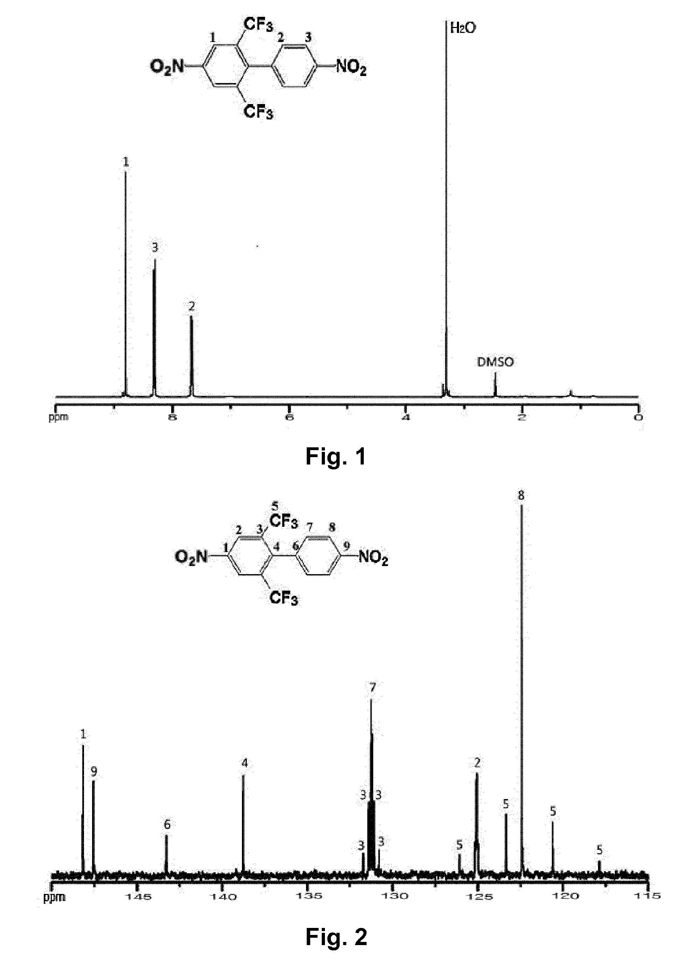

FIG. 1 is a ¹H NMR spectrum of a representative dinitro compound in one embodiment.
FIG. 2 is a ¹³C NMR spectrum of a representative dinitro compound in one embodiment.

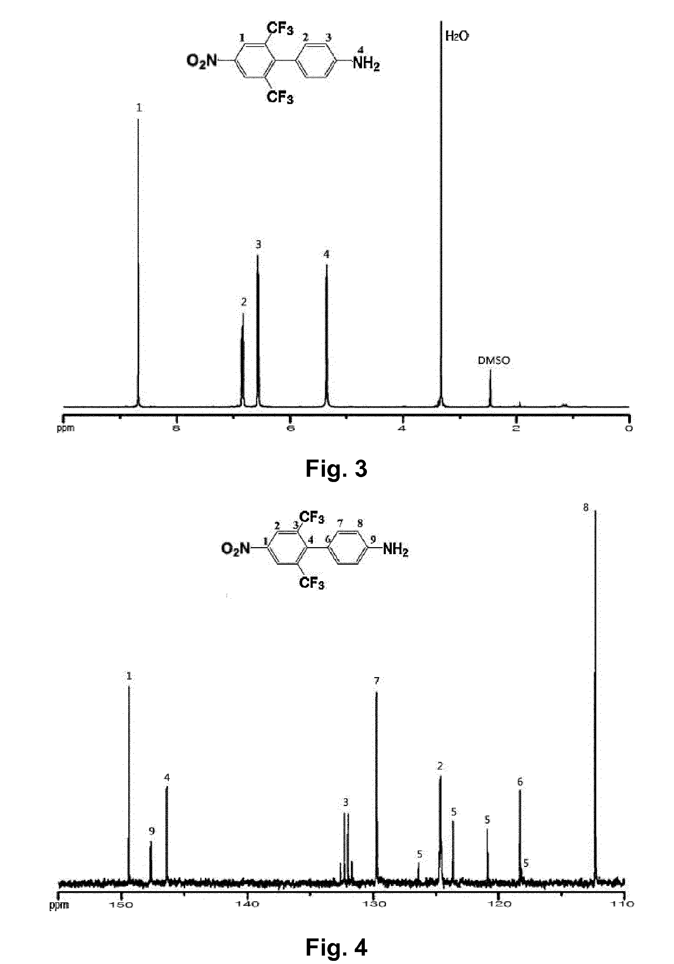

FIG. 3 is ¹H NMR spectrum of a representative mononitro compound in one embodiment.
FIG. 4 is ¹³C NMR spectrum of a representative mononitro compound in one embodiment.

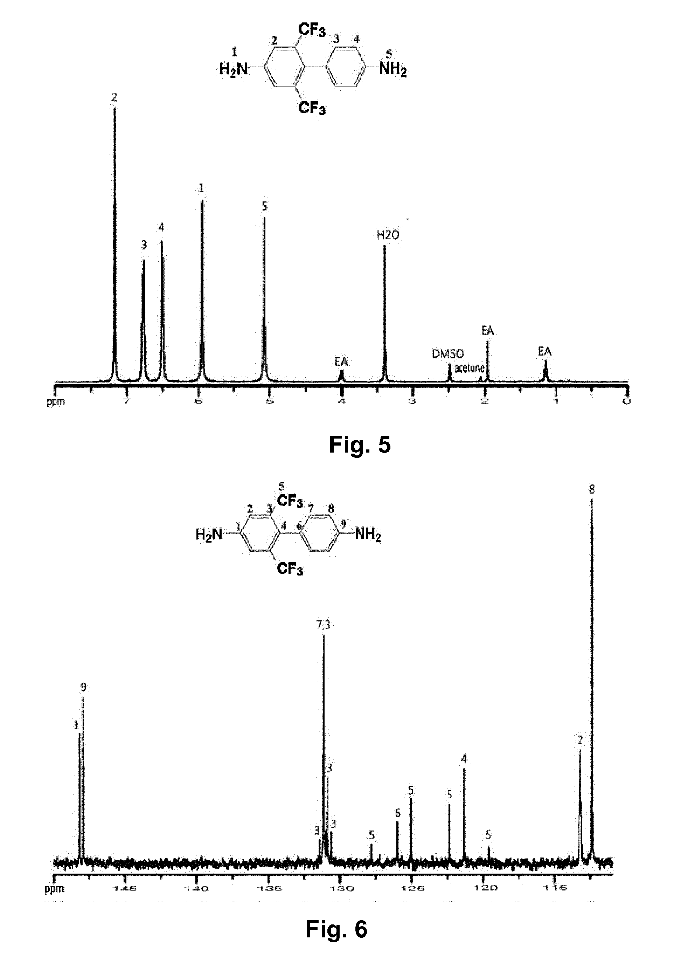

FIG. 5 is ¹H NMR spectrum of a representative diamine compound in one embodiment.
FIG. 6 is ¹³C NMR spectrum of a representative diamine compound in one embodiment

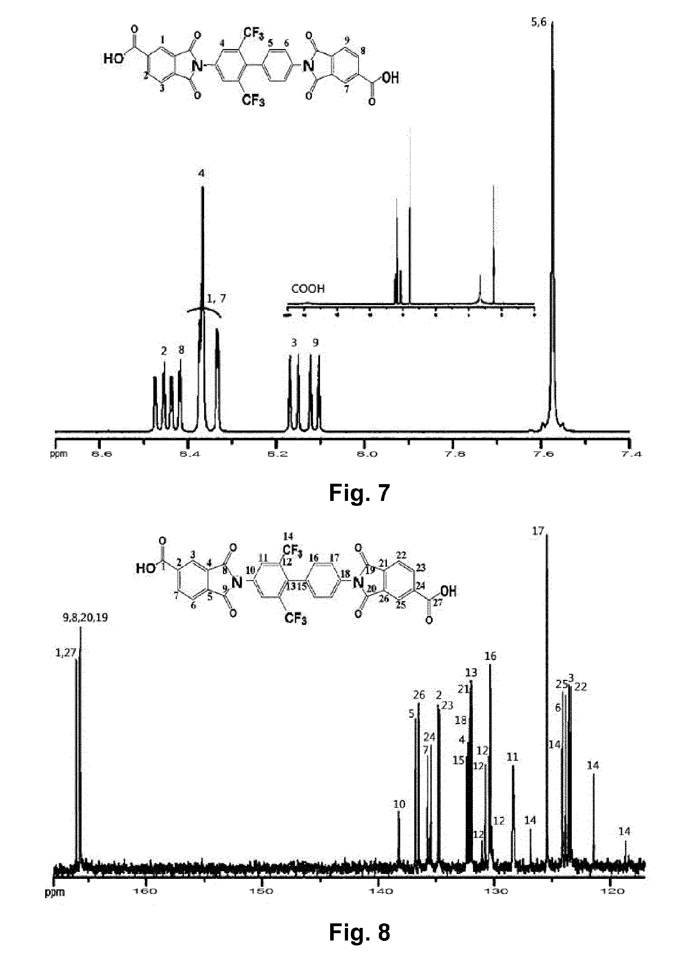

FIG. 7 is ¹H NMR spectrum of a representative dicarboxylic acid derivative in one embodiment.
FIG. 8 is ¹³C NMR spectrum of a representative dicarboxylic acid derivative in one embodiment.

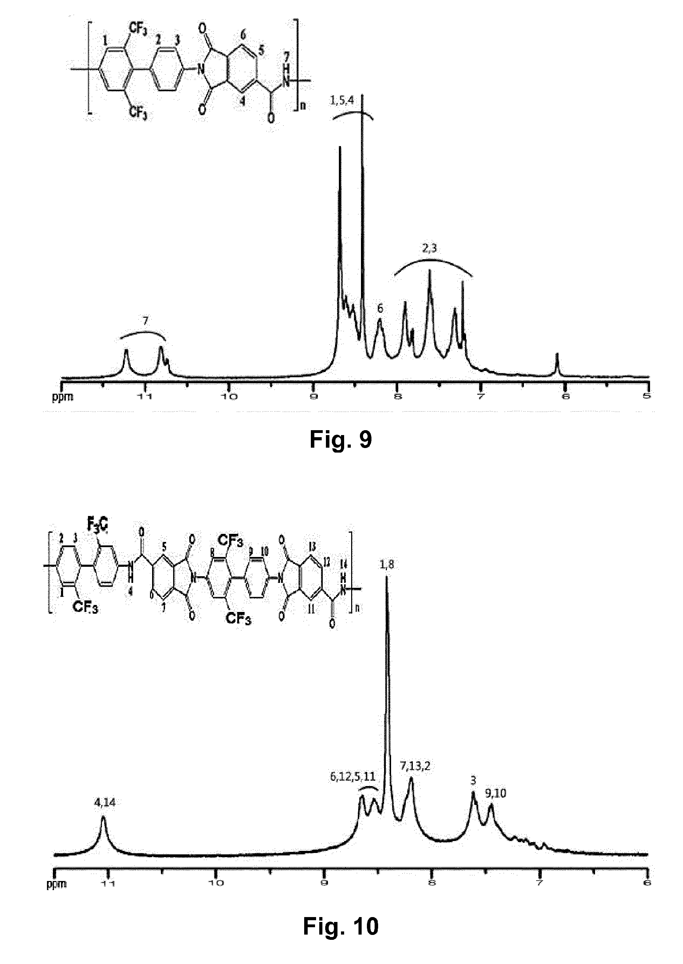

FIG. 9 is ¹H NMR spectrum of a representative polyamideimide(uuBTFB-PAI) in one embodiment.
FIG. 10 is ¹H NMR spectrum of a representative polyamideimide(suBTFB-PAI2) in one embodiment.

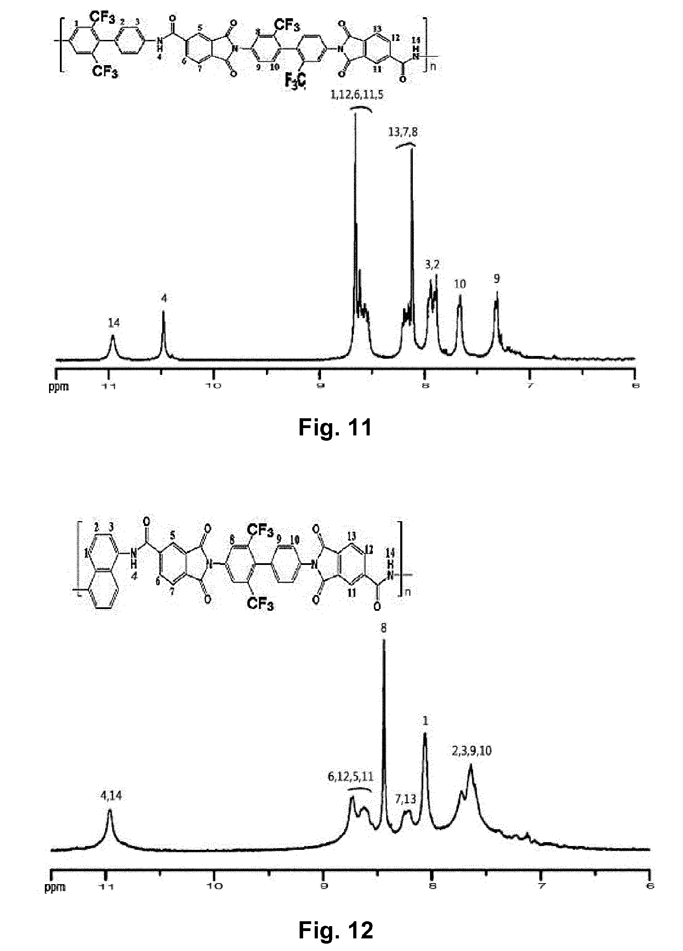

FIG. 11 is ¹H NMR spectrum of a representative polyamideimide(usBTFB-PAI) in one embodiment.
FIG. 12 is ¹H NMR spectrum of a representative polyamideimide(DAN-PAI) in one embodiment.

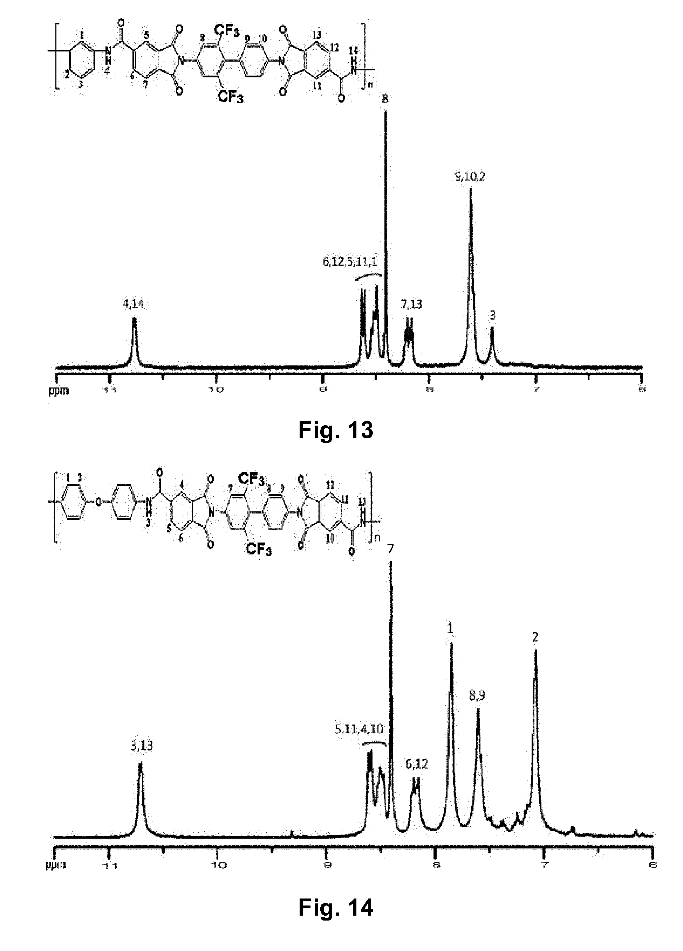

FIG. 13 is ¹H NMR spectrum of a representative polyamideimide(mPDA-PAI) in one embodiment.
FIG. 14 is ¹H NMR spectrum of a representative polyamideimide(ODA-PAI) in one embodiment.

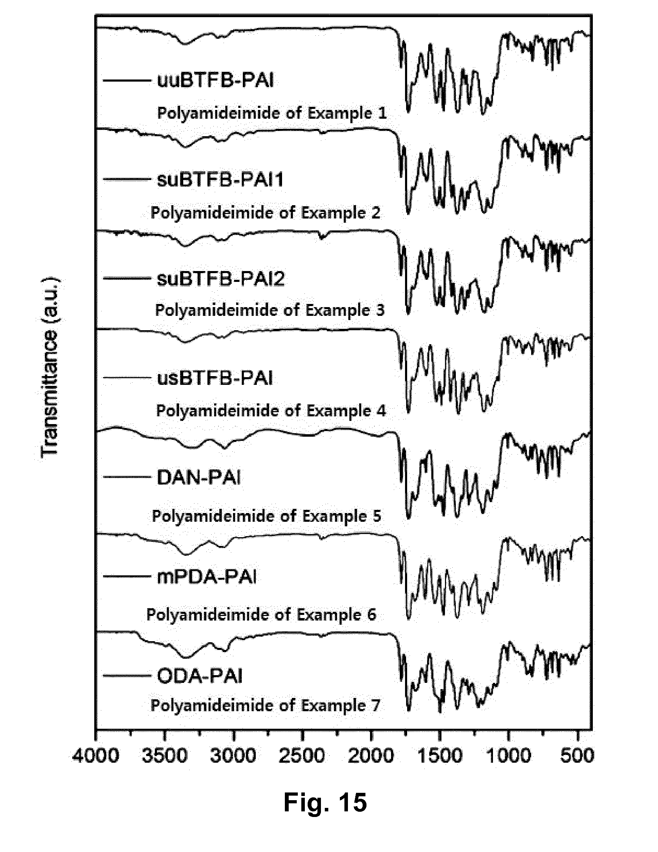

FIG. 15 is infrared (IR) spectra of polyamideimides from examples 1-7.

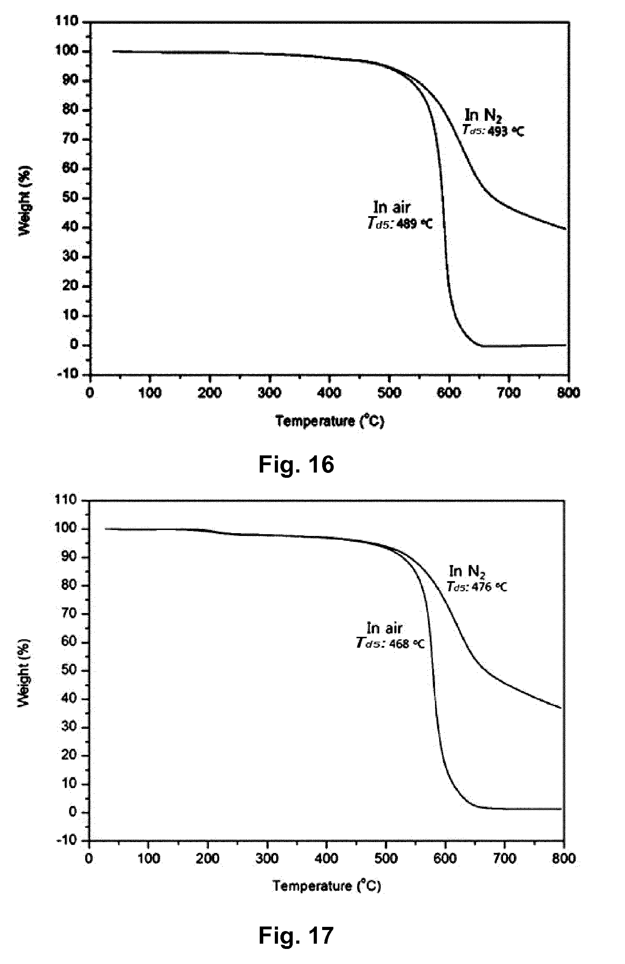

FIG. 16 is thermogravimetric analysis (TGA) of polyamideimide (uuBTFB-PAI) from example 1.
FIG 17 is is thermogravimetric analysis (TGA) of polyamideimide (suBTFB-PAI1) from example 2.

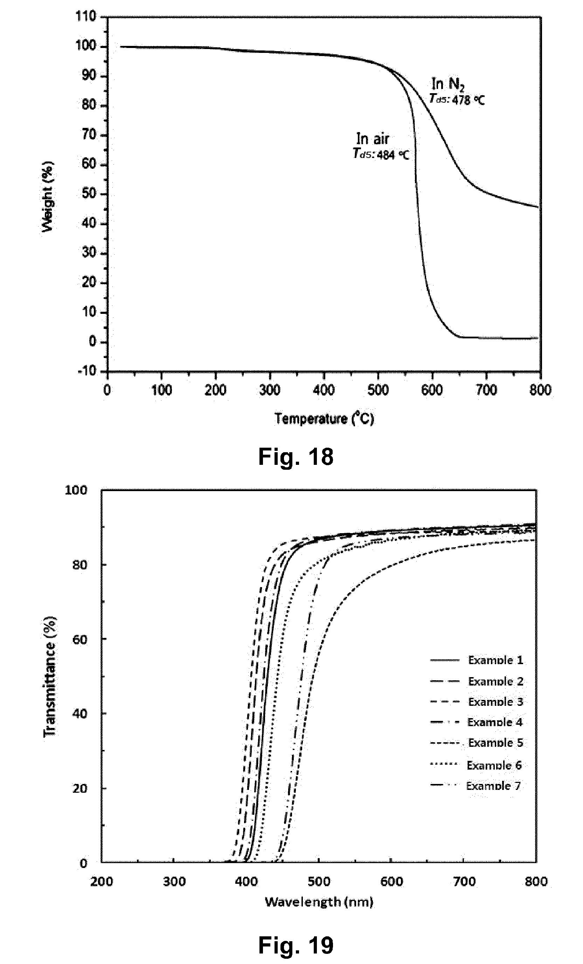

FIG 18 is is thermogravimetric analysis (TGA) of polyamideimide (suBTFB-PAI2) from example 3.
FIG 19 is UV-visible spectrum of polyamideimide from examples 1-7.

### Optimized Condition of the Present Invention

The below is the detailed explanation of the present invention.

Provides is an asymmetrical dicarboxylic acid derivative, as represented by Formula A, its alkali metal salt or its alkaline earth metal salt, wherein the two substituents, R and R' are attached to only cyclic group A of both cyclic group A and B of Formula A:

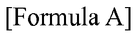

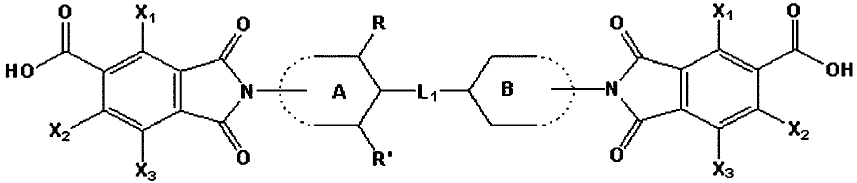

In said formula A,
wherein R, R', X1, X2 and X3 are identical to or different from each other, and are independently selected from the group consisting of C1 to C12 alkyl group unsubstituted or substituted with one or more halogen; C1 to C12 alkoxy group unsubstituted or substituted with one or more halogen; C2 to C12 alkenyl group unsubstituted or substituted with one or more halogen; C2 to C12 alkynyl group unsubstituted or substituted with one or more halogen; C4 to C30 cycloalkyl group unsubstituted or substituted with one or more halogen; C4 to C30 cycloalkenyl group unsubstituted or substituted with one or more halogen; C6 to C30 aryl group unsubstituted or substituted with one or more halogen, C1 to C12 alkyl group, C1 to C12 alkoxy group, C1 to C12 halogenated alkyl group and/or C1 to C12 halogenated alkoxy group; C3 to C30 heteroaryl group unsubstituted or substituted with one or more halogen, C1 to C12 alkyl group, C1 to C12 alkoxy group, C1 to C12 halogenated alkyl group and/or C1 to C12 halogenated alkoxy group; and C6 to C30 arylalkyl group unsubstituted or substituted with one or more halogen, C1 to C12 alkyl group, C1 to C12 alkoxy group, C1 to C12 halogenated alkyl group and/or C1 to C12 halogenated alkoxy group;
L1 represents a direct bond, -O-, -S-, -C(=O)O-, -OC(=O)-, -C(=O)-, -SO2-,-C(R1)(R2)-, -NR3, [58] where R1, R2 and R3 are identical to or different from each other, and are independently selected from the group consisting of hydrogen; C1 to C12 alkyl group unsubstituted or substituted with one or more halogen; C4 to C20 cycloalkyl group unsubstituted or substituted with one or more halogen; C6 to C20 aryl group unsubstituted or substituted with one or more halogen; and C3 to C20 heteroaryl group unsubstituted or substituted with one or more halogen;
said cyclic group 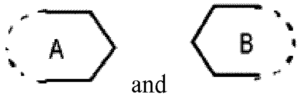 each independently represent C5 to C30 arylene or cycloalkylene unsubstituted or substituted with 5-membered or 6-membered ring which is unsubstituted or substituted or more halogen; or C5 to C30 heteroarylene or heterocycloalkylene unsubstituted or substituted with 5-membered or 6-membered ring which is unsubstituted or substituted or more halogen.

In one embodiment, the substituents R and R', which are characterized as bulky group, are introduced asymmetrically within the two cyclic groups of diamine, to inhibit several interactions polyimide or polyamide exhibits.

The said bulky group can be electron withdrawing group, and the said electron withdrawing group can be linear or branched perfluoroalkyl group. In one embodiment, an example of R and R' may be trifluoromethyl.

As mentioned above, the substitution with bulky and electron withdrawing substituent asymmetrically result in the enormous enhance of the solubility in organic solvent as well as transparency in form of film by lowering the interaction via cancelling out the symmetry of chain in the polyimide or polyamideimide.

In one embodiment, examples of R, R', X1, X2 and X3 may be independently selected from (i) C1 to C12 alkyl groups unsubstituted or substituted with one or more halogen, where C1 to C12 alkyl groups are linear or branched alkyl groups, such as but not limited to methyl, ethyl, n-propyl, i-propyl, n-butyl, i-butyl, or t-butyl, optionally substituted with one or more halogen(i.e., fluoride), such as trifluoromethyl or pentafluroethyl groups, and the like.

In another embodiment, examples of R, R', X1, X2 and X3 may be independently selected from (ii) C1 to C12 alkoxy groups unsubstituted or substituted with one or more halogen, where C1 to C12 alkoxy groups are linear or branched alkoxy groups, such as but not limited to methoxy, ethoxy, propoxy, or butoxy, optionally substituted with one or more halogen(i.e., fluoride), such as trifluoromethoxy, and pentafluoroethoxy groups, and the like.

In yet another embodiment, examples of R, R', X1, X2 and X3 may be independently selected from (iii) C1 to C12 alkenyl groups unsubstituted or substituted with one or more halogen, where C1 to C12 alkenyl groups are linear or branched alkenyl groups, such as but not limited to ethenyl, propenyl, or butenyl, optionally substituted with one or more halogen(i.e., fluoride), such as fluoroethenyl groups, and the like.

In yet still another embodiment, examples of R, R', X1, X2 and X3 may be independently selected from (iv) C1 to C12 alkynyl groups unsubstituted or substituted with one or more halogen, where C1 to C12 alkynyl groups are linear or branched alkynyl groups, such as but not limited to ethynyl, propynyl, or butynyl, optionally substituted with one or more halogen(i.e., fluoride), such as fluoroethynyl groups, and the like.

In one embodiment, examples of R, R', X1, X2 and X3 may be independently selected from (v) C4 to C30 cycloalkyl groups unsubstituted or substituted With one or more halogen, Where C4 to C30 are linear or branched cycloalkyl groups, such as cyclobutyl, cyclopentyl, cyclohexyl, and the like, optionally substituted with one or more halogen (i.e., fluoride), such as fluorocyclobutyl, and the like.

In another embodiment, examples of R, R', X1, X2 and X3 may be independently selected from (vi) C4 to C30 cycloalkenyl groups unsubstituted or substituted with one or more halogen, which are linear or branched cycloalkenyl groups, such as cyclopropenyl, cyclobutenyl, cyclopentenyl, cyclohexenyl, and the like, optionally substituted with one or more halogen (i.e., fluoride), such as fluorocyclobutenyl group, and the like.

In another embodiment, examples of R, R', X1, X2 and X3 may be independently selected from (vii) C6 to C30 aryl groups unsubstituted or substituted with one or more halogen, C1 to C12 alkyl, C1 to C12 alkoxy, C1 to C12 halogenated alkyl and/or C1 to C12 halogenated alkoxy group, such as phenyl, naphthyl, and the like..

In yet another embodiment, examples of R, R', X1, X2 and X3 may be independently selected from (viii) C3 to C30 heteroaryl groups unsubstituted or substituted with one or more halogen, C1 to C12 alkyl, C1 to C12 alkoxy, C1 to C12 halogenated alkyl and/or C1 to C12 halogenated alkoxy group, such as pyrrole, pyridyl, thiophenyl, indolyl, and the like optionally substituted with one or more halogen or linear or branched alkyl groups.

In yet still another embodiment, examples of R, R', X1, X2 and X3 may be independently selected from (ix) C6 to C30 arylalkyl groups unsubstituted or substituted with one or more halogen, C1 to C12 alkyl, C1 to C12 alkoxy, C1 to C12 halogenated alkyl and/or C1 to C12 halogenated alkoxy group, such as tolyl, mesityl, xylyl, and the like optionally substituted with one or more halogen or linear or branched alkyl groups.

The substituting group of the said R and R' may be a fluoroalkyl, fluoroalkoxy, or other substituted or unsubstituted aryl, preferably perfluoroalkyl, perfluoroalkoxy, or other substituted or unsubstituted phenyl group, and more preferably trifluoromethyl (-CF3) and pentafluoroethyl (-C2F5).

In the cases of cyclic groups A and B are 6-membered rings, the positions of amine of imide may be para-position with respect to the linker '-L-'.

In one embodiment, said substituents R and R' are identical to or different from each other, and are represent a hydrocarbyl group comprising fluorine.

Additionally, said '-L-' may be any one selected from the group consisting of a direct bond, -O-, or -S-.

In case of L1 is direct bond, the said R and R' represent C1 to C5 alkyl substituted with fluorine, and the said X1 to X3 are identical to or different from each other, and are selected from the group consisting of hydrogen, halogen, trifluromethyl, C1 to C5 alkyl, or C1 to C5 alkoxy, and the said cyclic group 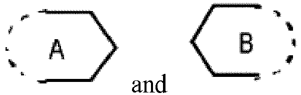 represent C6 to C12 arylene unsubstituted or substituted with one or more halogen.

More specifically, the present invention provides with dicarboxylic acid derivatives represented as Formula A-1, its alkali metal salt, or its alkaline earth metal salt: 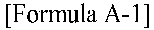 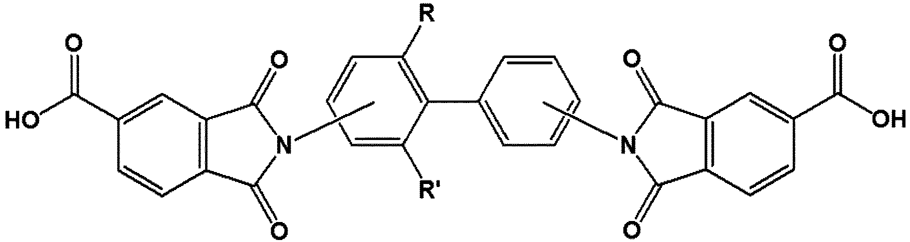
wherein the said R and R' are each CF3 or C2F5.

Asymmetric dicarboxylic acid of the present invention can be used as monomer of polyamideimide, possibly substituted with trifluoromethyl group in order to give better solubility in organic solvent, and control the structure in the aromatic polyamideimide and to provide with aromatic polyamideimide with good solubility, strength and adhesion.

In another embodiment, provided is a process for producing an asymmetric dicarboxylic acid derivatives represented as [formula A] in Reaction 1 used as monomer of polyamideimide.

The said Reaction 1, more specifically, represent the diamine compound represented as formula A-2 reacting with compound represented as formula A-3 in imidization reaction to give dicarboxylic acid derivatives represented as formula A. 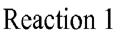 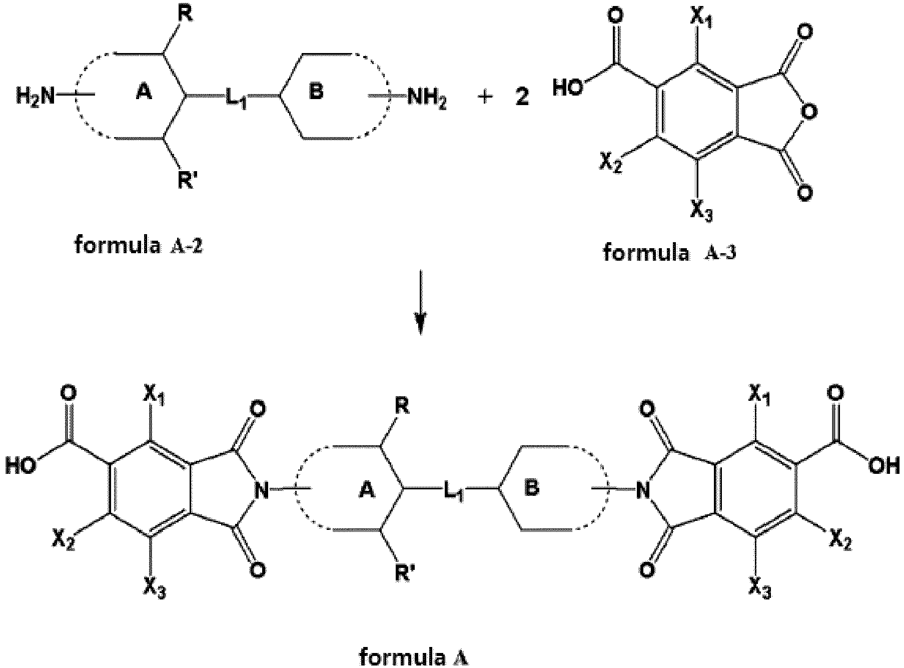 wherein R, R', X1 to X3, L1, and cyclic group 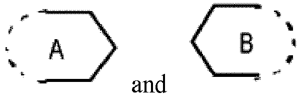 in the said Formula A, Formula A-2 and Formula A-3 are the same as defined as above.

The compound of formula A-2 can be synthesized by reaction between compound of formula 1 with compound of formula 2 as shown in Reaction A via nucleophilic reaction to give dinitro compound of formula 3, followed by hydrogenation of the said dinitro compound of formula 3.

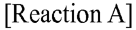

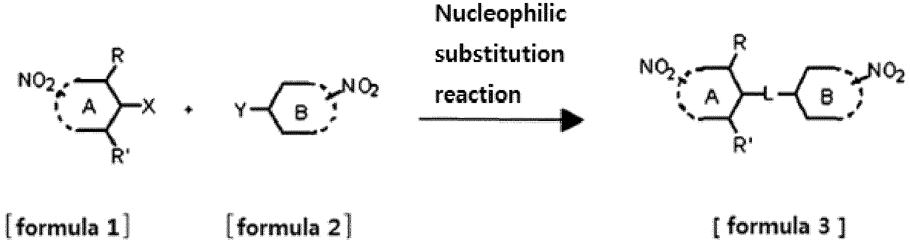

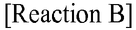

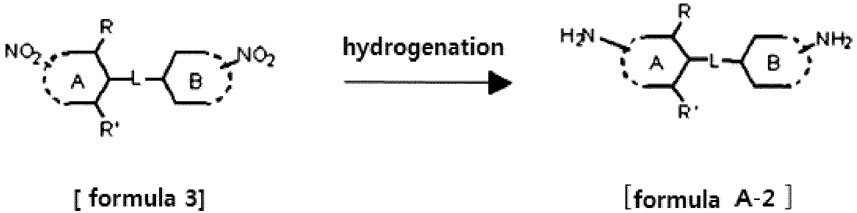

i) As shown in Reaction A or B, one of the X or Y may represent a halogen atom (-hal) such as F, Cl, Br, or l or ester, -C(O)-Cl, while the other may be a reactive functional group to form the linker -L- as in Formula 3 of the said Reaction A, such as OH, SH, NH or their alkali salt:

ii) wherein R, R', and cyclic group 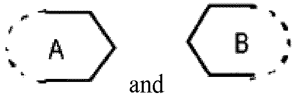 in the said Reaction A are the same as defined as above.

iii) the linker -L- is selected from a group consisting of -O-, -S-, -C(=O)O-, -OC(=O)-, -SO2-, and -NR3.

The synthesis of diamine compound of said formula A-2 can be processed according to [Reaction C].

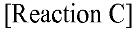

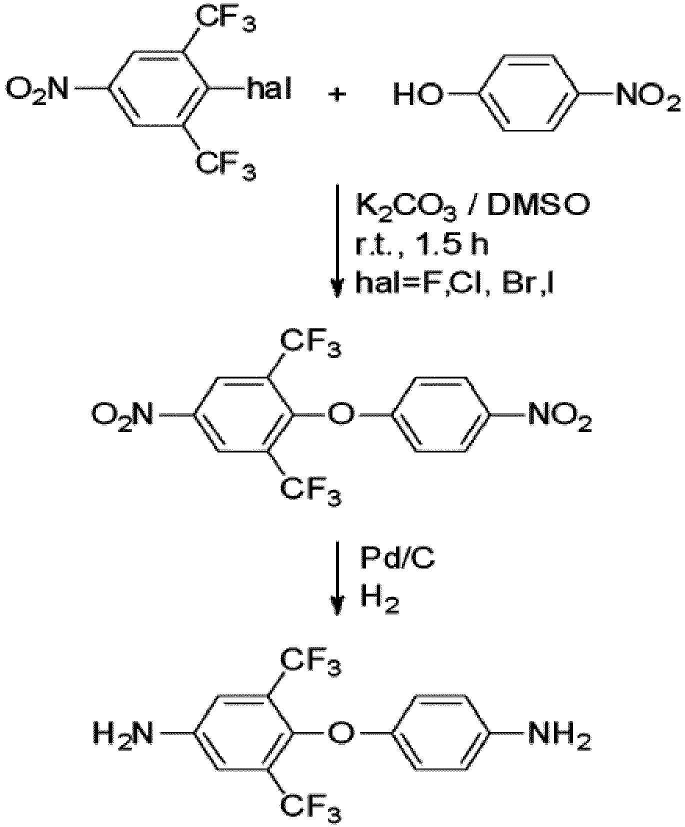

In the said [Reaction C], -X is hal, and Y is OH, and the linker L is -O-.

Meanwhile, in one embodiment, said dicarboxylic acid derivative of formula A is a monomer consisting of the said compound of formula A-2 with compound of formula A-3 in 2:1 ratio in mole. To reduce the side product such as compound of formula A-2 with compound of formula A-3 in 1:1 ratio in mole, the said compound of A-3 should be more than double of the compound of formula A-2 to obtain the said dicarboxylic acid of formula A.

In one embodiment, the imidization of said Reaction I in the synthesis of the said dicarboxylic acid derivative can be done via dehydration reaction in the presence of acid catalyst such as glacial acetic acid.

Also, the said imidization may need solvent in the synthesis of the said dicarboxylic acid.

The said solvent may be selected from a group consisting of N,N-dimethylformamide (DMF),

N,N-dimethyl acetamide (DMAc), N-methyl pyrrolidone (NMP), dimethyl sulfoxide (DMSO), and m-cresol and the like, and the reaction temperature is between 80 °C and 250 °C.

Also, the said imidization reaction is dehydration reaction, so that the removing water during reaction is critical by adopting water removing apparatus.

In general, the said imidization reaction involves with dissolving the starting material in the solvent, raising temperature for some time with stirring to complete imidation. For effective imidization reaction, small amount of dehydrating agent or imidization catalyst is added continuously during the reaction to remove water, resulting in the higher degree of imidization. The dehydrating agent or imidization catalyst may be any dehydrating agent or imidization catalyst known to a person skilled in the art. After the completion of the imidization reaction, the reaction mixture is added to an excess amount of a mixture of methanol and water to form precipitates, which are then washed with hot water and alcohol, followed by drying in a vacuum oven.

The present invention also provides with the diamine represented as [formula D] wherein two substitutents R and R' are attached to the one side of cyclic A asymmetrically.

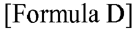

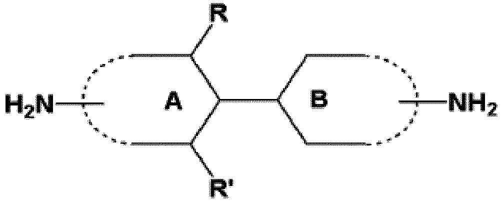

wherein R, R', and cyclic group 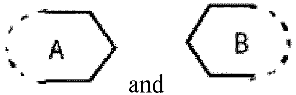 in the said Formula D are the same as defined as above.

More specifically, said substituents R and R' of diamine are identical to or different from each other, and are hydrocarbyl group comprising fluorine.

In this case, said R and R' are C1 to C5 alkyl substituted with fluorine, and the said cyclic group 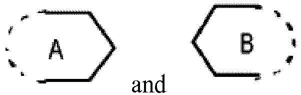 represent C6 to C12 arylene unsubstituted or substituted with one or more halogen.

In one embodiment, the diamine represented as the said [formula D] of the present invention may be the compound represented as formula D-1. 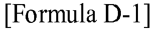 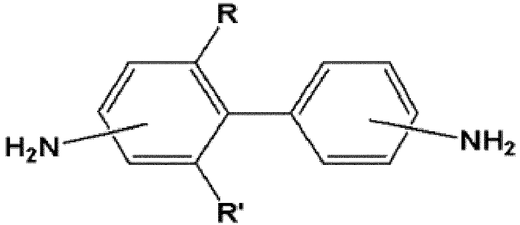 whereas R and R' may independently represent CF3 or C2F5.

The said diamine of formula D-1 has trifluoromethyl or pentafluoroethyl substituted on aromatic ring, which inhibits several interactions of polyimide, enhancing the solubility of polyamideimide, as well as lowering the moisture absorption, dielectric constant and refractive index. Also trifluromethyl or pentafluorethyl group which is substituted asymmetrically within the molecule cancel out the symmetry in chains of polyamideimie, therefore weakening the interaction and enhancing solubility toward organic solvent.

The present invention provides with the process for producing a diamine represented as said formula D.

Said process for producing the diamine represented as formula D comprising steps of reacting nitro compound of formula D-2 and amine compound of formula D-3 or nitro compound of formula D-4 via Suzuki coupling to give either mononitro compound of formula D-5 or dinitro compound of formula D-6 shown in reaction 5; and b) reducing either mononitro compound of formula D-5 or dinitro compound of formula D-6 via Reaction 6, reducing nitro group to amine.

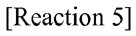

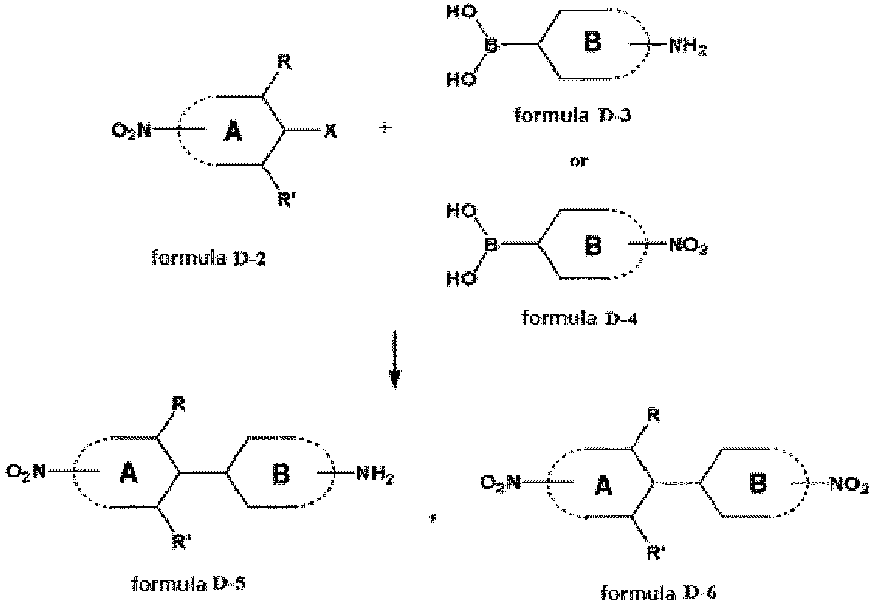

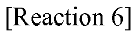

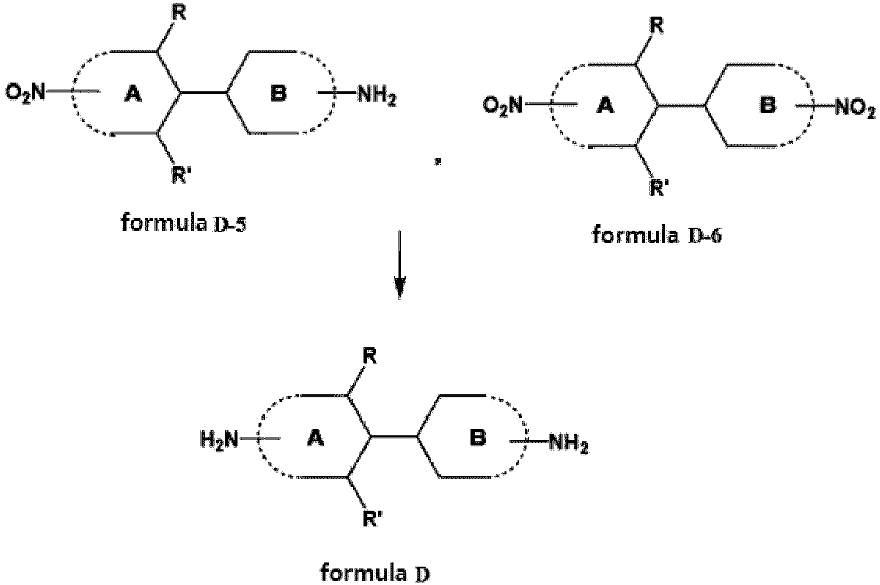

Wherein R, R' and cyclic group 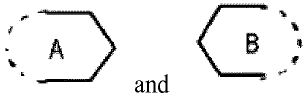 are the same as defined as above, and X is halogen selected from I, Br, and Cl.

The said Reaction 5 of the present invention, Suzuki coupling, uses palladium complex catalyst, more specifically tetrakistriphenylphosphine palladium complex, and the solvent may be aromatic hydrocarbon such as benzene or toluene, or alcohol such as methanol, ethanol and etc.

Also, in the said reaction 6, compound responsible for the reducing nitro group to amine group can be any chemicals, but preferably hydrogen and palladium catalyst, SnCl2 and etc.

In one embodiment, provided is a polyamideimide represented as [formula B] by reacting the said dicarboxylic acid with known diamine in the condensation thru the amidation reaction.

Also, the present invention provides with a polyamideimide represented as [formula C] by reacting trimelitic anhydride with known diamine compound in imidization reaction to give dicarboxylic acid derivatives, followed by reacting the said dicarboxylic acid derivatives with the asymmetric diamine compound represented as formula D in condensation.

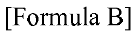

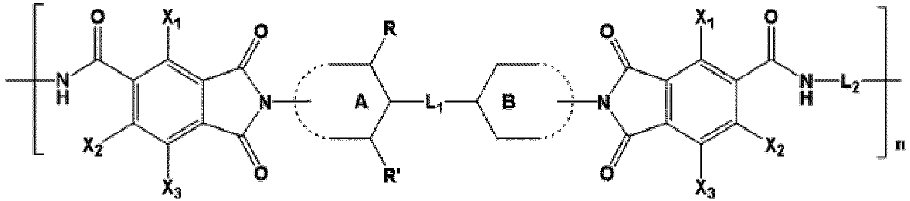

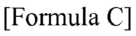

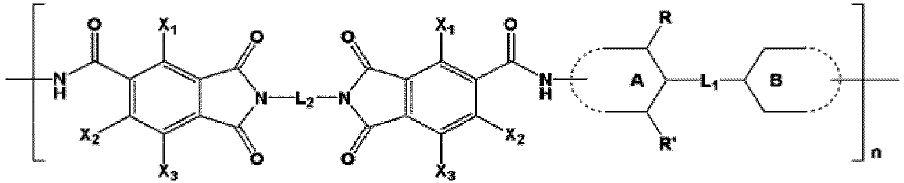

In said [Formula B] and [Formula C],

R, R', X1 to X3, L1, and cyclic group 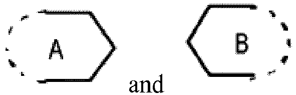 are the same as defined as in Formula A, and L2 is selected from the group consisting of C1 to C20 alkylene group unsubstituted or substituted with one or more halogen; C6 to C20 arylene group unsubstituted or substituted with one or more halogen; C6 to C30 arylene group unsubstituted or substituted with C1 to C12 alkyl group, C1 to C12 alkoxy group, C1 to C12 halogenated alkyl group, C1 to C12 halogenated alkoxy group; C2 to C20 heteroarylene group unsubstituted or substituted with one or more halogen; C4 to C20 cycloalkylene group unsubstituted or substituted with one or more halogen; and n is an integer selected from 10 to 5,000,000.

More specifically, R and R' of said formula B and formula C are identical to or different from each other, and are independently represent a hydrocarbyl group comprising fluorine, and said L1 may be selected from a group consisting of direct bond, -O-, and -S-.

In one embodiment, L1 is direct bond, L2 is selected from a group consisting of C1 to C12 alkylene, C6 to C12 arylene, C6 to C16 arylene substituted with C1 to C12 halogenated alkyl, C2 to C12 heteroarylene, and R and R' may independently represent C1 to C5 alkyl substituted with fluorine,
said X1 to X3 are identical to or different from each other, and are independently selected from a group consisting of hydrogen, halogen, trifluoromethyl, C1 to C5 alkyl, C1 to C5 alkoxyl, and cyclic group 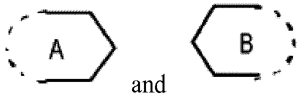 each represent C6 to C12 arylene unsubstituted or substituted with one or more halogen.

Also in case of cyclic groups A and B in polyamideimide of the present invention being as 6-membered rings, the positions of amine or amide groups may be para- to the linker '-L-'.

In the present invention, the said L2 may be selected from a group consisting of

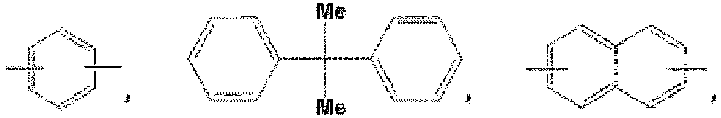

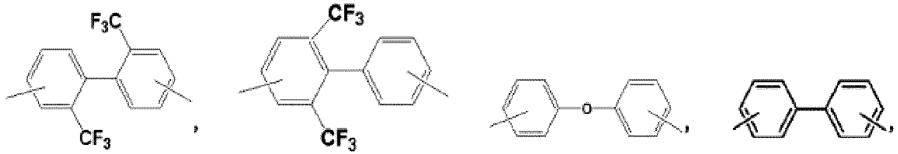

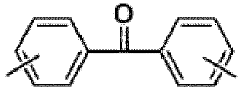

In one embodiment, polyamideimide of the present invention is either compounds represented as Formula B-1 or C-1. 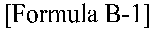 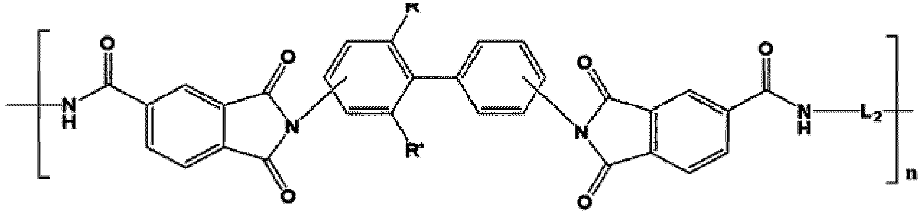 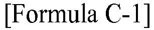 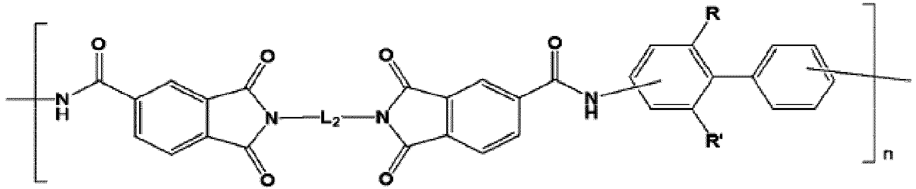 said R and R' each independently represent CF3 or C2F5, and L2 may be selected from a group consisting of

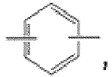

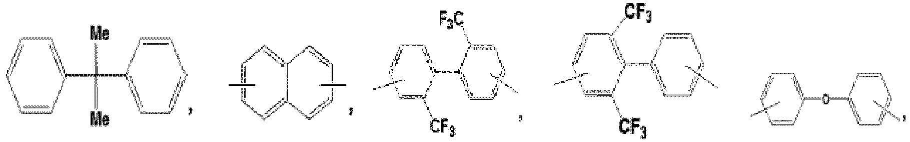

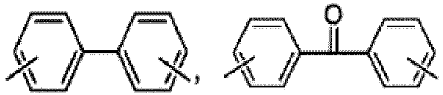

Also, the present invention provides with a process for producing a polyamideimide represented as formula B via amidation between asymmetrical dicarboxylic acid derivative represented as formula A and diamine monomer represented as formula B-2 in Reaction 2.

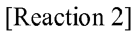

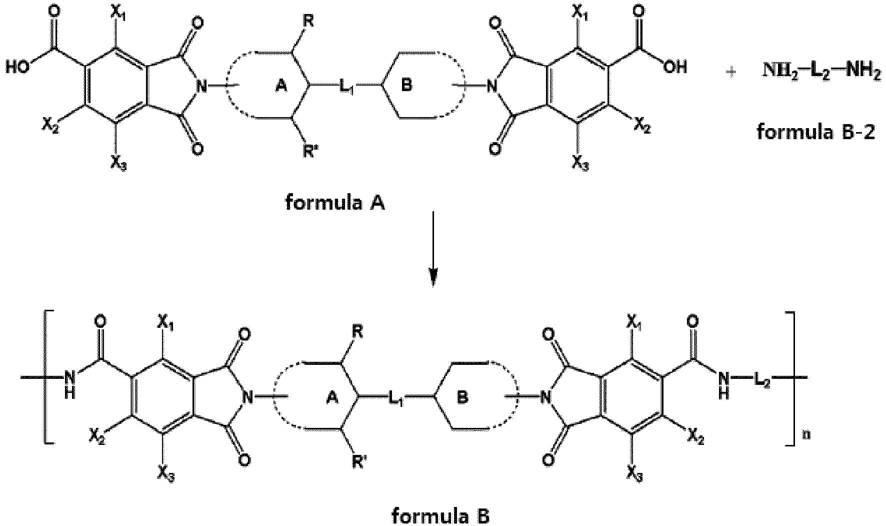

In said Reaction 2, R, R', X1 to X3, L1, L2, n and cyclic group 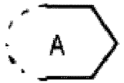 and 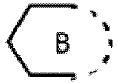 are the same as defined as above.

The said amidation condensation reaction can be done by solving the diamine monomer of formula B-2 with dicarboxylic acid derivatives monomer in the organic solvent while mixing.

The solvent may be selected from sulfoxide or sulfone solvents such as dimethyl sulfoxide, diphenyl sulfone, tetra methyl sulfone; amide solvents such as N,N-dimethyl acetamide(DMAc), N,N'-diethyl acetamide, N-methyl-2-pyrrolidone(NMP), γ-butyl lactone, hexamethyl phosphoamide; halogenated alkyl solvent such as chloroform, methylene chloride; ether solvent such as tetrahydrofuran, 1,4-dioxane, p-cresolmethylether. The solvent may be used individually or in mixture of two or more kinds, in more preferably, may be selected from a group consisting of N,N-dimethylformaldehyde, N,N- dimethyl acetamide(DMAc), N-methyl-2-pyrrolidone(NMP) and dimethylsulfoxide (DMSO). Also the reaction temperature in amidation condensation reaction is between 60 and 250 °C, more preferably between 80 and 200 °C, with the reaction time of 30 min to 20 hours mixing.

Also, the said amidation reaction is dehydration reaction, so that the removing water during reaction is critical by adopting water removing apparatus.

The concentration of solvent is normally 5-20 %(weight of reactants(g)/amount of solvent(ml)) in amidation reaction and the temperature is raised and mixed during certain period of time to complete amidation. For effective amidation reaction, small amount of dehydrating agent or imidization catalyst is added continuously during the reaction to remove water, resulting in the higher degree of the amidation. The dehydrating agent may be any dehydrating agent known to a person skilled in the art. After the completion of the amidation reaction, the reaction mixture is added to an excess amount of a mixture of methanol and water to form precipitates, which are then washed with hot water and alcohol, followed by drying in a vacuum oven.

Also the present invention provides with a process for producing the polyamideimide represented as Formula C comprising steps of a) imidization condensation between trimelitic anhydride represented as formula A-3 and diamine monomer represented as formula B-2 to give dicarboxylic acid derivatives of formula C-3, in Reaction 3 and b) amidation condensation between dicarboxylic acid derivative of formula C-3 with diamine compound of formula A-2 in Reaction 4;

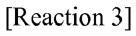

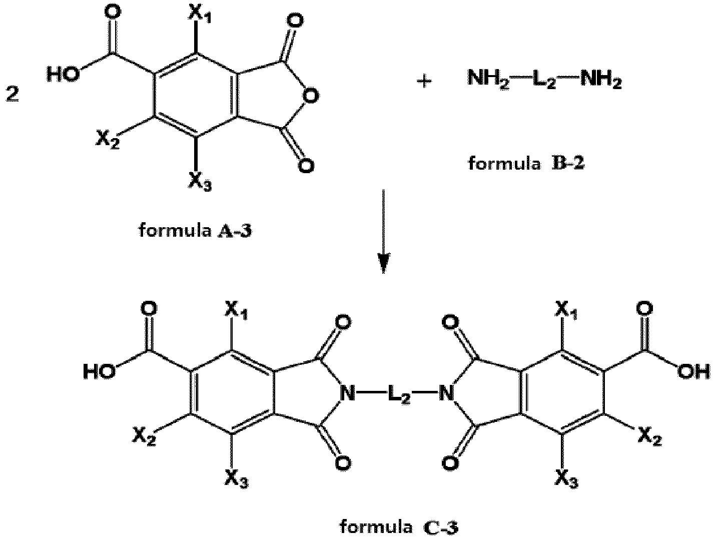

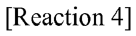

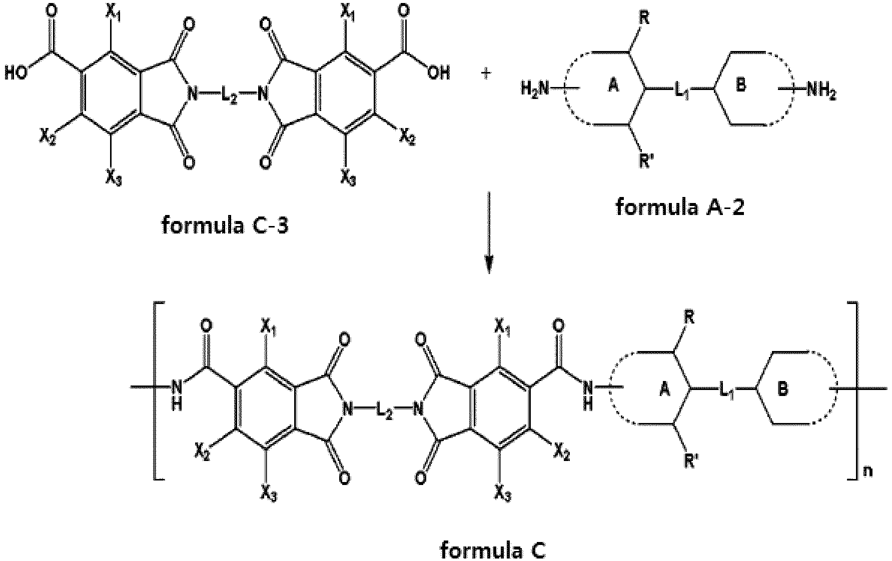

In Reaction 3 and Reaction 4

R, R', X1 to X3, L1, L2, n and cyclic group 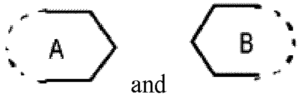 are the same as defined as above.

Imidization and amidation reaction of Reaction 3 and 4 can be done under the same reaction condition as the reaction condition of imidization reaction of said dicarboxylic acid derivatives and amidation reaction of polyimideamide.

Also, in the process for producing the polyamideimide of the present invention, the diamine represented as formula B-2 is selected from a group consisting of 2,2'-bis(trifluoromethyl)benzidine, 2,6-bis(trifluoromethyl)benzidine, p-phenylenediamine, m-phenylenediamine, p-aminobenzylamine, m-aminobenzylamine, 4,4'-diaminodiphenylmethane, 3,4'-diaminodiphenylmethane, 3,3'-diaminodiphenylmethane, 4,4'-diaminodiphenylethane, 4,4'-diaminobenzanilide, 4,4'-diaminodiphenyl ether, 3,4'-diaminodiphenyl ether, 3,3'-diaminodiphenyl ether, 2,4'-diaminodiphenyl ether, 2,2'-diaminodiphenyl ether, 2,3'-diaminodiphenyl ether, 1,4-bis(4-aminophenoxy)benzene, 1,4-bis(3-aminophenoxy)benzene, 1,3-bis(4-aminophenoxy)benzene, 1,3-bis(3-aminophenoxy)benzene, 2,2-bis[4-(4-aminophenoxy)phenyl)]propane, 2,2-bis[4-(3-aminophenoxy)phenyl)]propane, bis(4-aminophenyl)sulfide, bis(3-aminophenyl)sulfide, 3,4-diaminophenyl sulfide, bis(4-aminophenyl)sulfoxide, bis(3-aminophenyl)sulfoxide, 3,4-diaminophenyl sulfoxide, bis(4-aminophenyl)sulfone, bis(3-aminophenyl)sulfone, 3,4-diaminophenyl sulfone, 4,4'-diaminobenzophenone, 3,4'-diaminobenzophenone, 3,3'-diaminobenzophenone, bis[4-(4-aminophenoxy)phenyl]sulfone, bis[4-(3-aminophenoxy)phenyl]sulfone, bis[4-(4-aminophenoxy)phenyl]ether, 1,4-bis[4-(3-aminophenoxy)benzoyl]benzene, 1,3-bis[4-(3-aminophenoxy)benzoyl]benzene, 4,4'-bis[3-(4-aminophenoxy)benzoyl]diphenyl ether, 4,4'-bis[3-(3-aminophenoxy)benzoyl]diphenyl ether, 4,4'-bis[4-(4-amino-α,α-dimethylbenzyl)phenoxy]benzophenone, 4,4'-bis[4-(4-amino-α,α-dimethylbenzyl)phenoxy]diphenyl sulfone, bis[4-{4-(4-aminophenoxy)phenoxy}phenyl]sulfone, 1,4-bis[4-(4-aminophenoxy)-α,α-dimethylbenzyl]benzene, 1,3-bis[4-(4-aminophenoxy)-α,α-dimethylbenzyl]benzene, 2,2-bis[4-(4-aminophenoxy)phenyl]-1,1,1,3,3,3-hexafluoropropane, 2,2-bis[4-(3-aminophenoxy)phenyl]-1,1,1,3,3,3-hexafluoropropane, 1,3-diaminonaphthalene, 1,4-diamino naphthalene, 1,5-diaminonaphthalene, 2,6-diaminonaphthalene, 2,2'-dimethyl-4,4'-diaminobiphenyl, 3,3'-dimethyl-4,4'-diaminobiphenyl, 2,2'-ditrifluoromethyl-4,4'-diaminobiphenyl, 3,3'-ditrifluoromethyl-4,4'-diaminobiphenyl, 5-amino-1-(4'-aminophenyl)-1,3,3-trimethylindane,
6-amino-1-(4'-aminophenyl)-1,3,3-trimethylindane, 2,2-bis[4-(4-aminophenoxy)phenyl)]propane, 2,2-bis[4-(4-aminophenoxy)phenyl)]hexafluoropropane, 2,2-bis(4-aminophenyl)hexafluoropropane, 2,2-bis[4-(4-aminophenoxy)phenyl]sulfone, 1,4-bis(4-aminophenoxy)benzene, 1,3-bis(4-aminophenoxy)benzene, 1,3-bis(3-aminophenoxy)benzene, 9,9-bis(4-aminophenyl)-10-hydroanthracene, 2,7-diaminofluorene, 9,9-dimethyl-2,7-diaminofluorene, 9,9-bis(4-aminophenyl)fluorene, 4,4'-methylene bis(2-chloroaniline), 2,2',5,5'-tetrachloro-4,4'-diaminobiphenyl, 2,2'-dichloro-4,4'-diamino-5,5'-dimethoxybiphenyl, 3,3'-dimethoxy-4,4'-diaminobiphenyl, 4,4'-(p-phenyleneisopropylidene)bisaniline, 4,4'-(m-phenyleneisopropylidene)bisaniline, 2,2'-bis[4-(4-amino-2-trifluoromethylphenoxy) phenyl]hexafluoropropane, 4,4'-diamino-2,2'-bis(trifluoromethyl)biphenyl, 4,4'-bis[(4-amino-2-trifluoromethyl)phenoxy]-octafluorobiphenyl.

Also, in the process for producing the polyamideimide of the present invention, the diamine compound represented as formula B-2 is mixed with diamine different from diamine of formula B-2, said different diamine is any one selected from the group consisting of 2,2'-bis(trifluoromethyl)benzidine, 2,6-bis(Trifluoromethyl)benzidine, p-phenylenediamine, m-phenylenediamine, p-aminobenzylamine, m-aminobenzylamine, 4,4'-diaminodiphenylmethane, 3,4'-diaminodiphenylmethane, 3,3'-diaminodiphenylmethane, 4,4'-diaminodiphenylethane, 4,4'-diaminobenzanilide, 4,4'-diaminodiphenyl ether, 3,4'-diaminodiphenyl ether, 3,3'-diaminodiphenyl ether, 2,4'-diaminodiphenyl ether, 2,2'-diaminodiphenyl ether, 2,3'-diaminodiphenyl ether, 1,4-bis(4-aminophenoxy)benzene, 1,4-bis(3-aminophenoxy)benzene, 1,3-bis(4-aminophenoxy)benzene, 1,3-bis(3-aminophenoxy)benzene, 2,2-bis[4-(4-aminophenoxy)phenyl]propane, 2,2-bis[4-(3-aminophenoxy)phenyl]propane, bis(4-aminophenyl)sulfide, bis(3-aminophenyl)sulfide, 3,4-diaminophenyl sulfide, bis(4-aminophenyl)sulfoxide, bis(3-aminophenyl)sulfoxide, 3,4-diaminophenyl sulfoxide, bis(4-aminophenyl)sulfone, bis(3-aminophenyl)sulfone, 3,4-diaminophenyl sulfone, 4,4'-diaminobenzophenone, 3,4'-diaminobenzophenone, 3,3'-diaminobenzophenone, bis[4-(4-aminophenoxy)phenyl]sulfone, bis[4-(3-aminophenoxy)phenyl]sulfone, bis[4-(4-aminophenoxy)phenyl] ether, 1,4-bis[4-(3-aminophenoxy)benzoyl]benzene, 1,3-bis[4-(3-aminophenoxy)benzoyl]benzene, 4,4'-bis[3-(4-aminophenoxy)benzoyl]diphenyl ether, 4,4'-bis[3-(3-aminophenoxy)benzoyl]diphenyl ether, 4,4'-bis[4-(4-amino-α,α-dimethylbenzyl)phenoxy]benzophenone, 4,4'-bis[4-(4-amino-α,α-dimethylbenzyl)phenoxy]diphenyl sulfone, bis[4-{4-(4-aminophenoxy)phenoxy}phenyl]sulfone, 1,4-bis[4-(4-aminophenoxy)-α,α-dimethylbenzyl]benzene, 1,3-bis[4-(4-aminophenoxy)-α,α-dimethylbenzyl]benzene, 2,2-bis[4-(4-aminophenoxy)phenyl]-1,1,1,3,3,3-hexafluoropropane, 2,2-bis[4-(3-aminophenoxy)phenyl]-1,1,1,3,3,3-hexafluoropropane, 1,3-diaminonaphthalene, 1,4-diamino naphthalene, 1,5-diaminonaphthalene, 2,6-diaminonaphthalene, 2,2'-dimethyl-4,4'-diaminobiphenyl, 3,3'-dimethyl-4,4'-diaminobiphenyl, 2,2'-ditrifluoromethyl-4,4'-diaminobiphenyl, 3,3'-ditrifluoromethyl-4,4'-diaminobiphenyl, 5-amino-1-(4'-aminophenyl)-1,3,3-trimethylindane, 6-amino-1-(4'-aminophenyl)-1,3,3-trimethylindane, 2,2-bis[4-(4-aminophenoxy)phenyl]propane, 2,2-bis[4-(4-aminophenoxy)phenyl]hexafluoropropane, 2,2-bis(4-aminophenyl)hexafluoropropane, 2,2-bis[4-(4-aminophenoxy)phenyl]sulfone, 1,4-bis(4-aminophenoxy)benzene, 1,3-bis(4-aminophenoxy)benzene, 1,3-bis(3-aminophenoxy)benzene, 9,9-bis(4-aminophenyl)-10-hydroanthracene, 2,7-diaminofluorene, 9,9-dimethyl-2,7-diaminofluorene, 9,9-bis(4-aminophenyl)fluorene, 4,4'-methylene-Bis(2-chloroaniline), 2,2',5,5'-tetrachloro-4,4'-diaminobiphenyl, 2,2'-dichloro-4,4'-diamino-5,5'-dimethoxybiphenyl, 3,3'-dimethoxy-4,4'-diaminobiphenyl, 4,4'-(p-phenyleneisopropylidene)bisaniline, 4,4'-(m-phenyleneisopropylidene)bisaniline, 2,2'-bis[4-(4-amino-2-trifluoromethylphenoxy)phenyl]hexafluoropropane, 4,4'-diamino-2,2'-bis(trifluoromethyl)biphenyl, 4,4'-bis[(4-amino-2-trifluoromethyl)phenoxy]-octafluorobiphenyl, 1,1-methaxylylenediamine, 1,3-propanediamine, tetramethylenediamine, pentamethylenediamine, hexamethylenediamine, heptamethylenediamine, octamethylenediamine, nonamethylenediamine, 1,4-diaminocyclohexane, isophoronediamine, tetrahydrodicyclopentadienylenediamine, hexahydro-4,7-methanoindanylenedimethylenediamine, tricyclo[6,2,1,02.7]-undecyclenedimethyldiamine, 4,4'-methylenebis(cyclohexylamine), 2,3-diaminopyridine, 2,6-diaminopyridine, 3,4-diaminopyridine, 2,4-diaminopyrimidine, 5,6-diamino-2,3-dicyanopyrazine, 5,6-diamino-2,4-dihydroxypyrimidine, 2,4-diamino-6-dimethylamino-1,3,5-triazine, 1,4-bis(3-aminopropyl)piperazine, 2,4-diamino-6-isopropoxy-1,3,5-triazine, 2,4-diamino-6-methoxy-1,3,5-triazine, 2,4-diamino-6-phenyl-1,3,5-triazine, 2,4-diamino-6-methyl-s-triazine, 2,4-diamino-1,3,5-triazine, 4,6-diamino-2-vinyl-s-triazine, 2,4-diamino-5-phenylthiazole, 2,6-diaminopurine, 5,6-diamino-1,3-dimethyluracil, 3,5-diamino-1,2,4-triazole, 6,9-diamino-2-ethoxyacridine lactate, 3,8-diamino-6-phenylphenanthridine, 1,4-diaminopiperazine, 3,6-diaminoacridine, bis(4-aminophenyl)phenylamine, 3,6-diaminocarbazole, N-methyl-3,6-diaminocarbazole, N-ethyl-3,6-diaminocarbazole, N-phenyl-3,6-diaminocarbazole, N,N'-di(4-aminophenyl)-benzidine.

Meanwhile, the present invention provides a film which is prepared by dissolving polyamideimide according to present invention in polar aprotic organic solvent or phenolic solvent, followed by drying the said solvent.

The polar aprotic organic solvent may be selected from a group consisting of N,N-dimethylformamide (DMF), N,N-dimethyl acetamide (DMAc), N-methyl pyrrolidone (NMP), dimethyl sulfoxide (DMSO), and tetrahydrofuran (THF), phenolic solvent is phenol, o-cresol, m-cresol, p-cresol, more preferably DMAc.

### EXAMPLE

The present invention will be understood more clearly from the Examples outlined below, and are not meant to limit the scope of the invention. Simple modifcations of the present invention may be accomplished by a person having ordinary skill in the art, and as such any of these modifications are included in the present invention.

The following method may be used to determine the structure and properties of monomer and polymers according to examples.

Infrared Spectrum(IR) and Nuclear Magnetic Resonance (NMR)

The structure of the synthesized material was determined by IR (UV spectroscopy) and NMR. IR spectra was obtained from potassium bromide (KBr) or thin film using a Bruker EQUINOX-55 spectrophotometer, and NMR spectra was obtained by dissolving compounds in chloroform, dimethyl sulfoxide-d6, then using a Bruker Fourier Transform AVANCE 400 spectrometer.

Inherent Viscosity

Inherent viscosity of the synthesized polymer is determined by dissolving the polymer in N-methylpyrrolidone(NMP) in the concentration of 0.5 g/dL, followed by using Ubbelohde type viscometer at 30 °C.

Thermal Stability Analysis

Thermogravimetric Analysis (TGA), Differential Scanning Calorimetry (DSC), and Thermo mechanical

Analysis (TMA) were measured using TA TGA Q500, DSC Q100, and TMA 2940 instruments, respectively. TGA and DSC in the case of 10 °C/min rate of increase of temperature was measured by, TMA in the case of 5 °C/min rate of increase of temperature was measured. The thermal analysis, all measured under a constant nitrogen flow, TGA analysis was performed under a constant air flow. Temperatures of 5% and 10% weight loss were measured from the TGA analysis, the glass transition temperature(Tg) was chosen by selecting the middle part where there was a change in the slope of the curve, and the coefficient of thermal expansion (CTE) was measured using TMA in the temperature range between 50 and 250 °C.

Transparency and Refractive Index

UV-visible spectra is taken in transmittance mode using film with thickness of 60-80 *µ*m. The refractive index was determined by a Sairon SPA-4000 prism coupler using a 630 and 1310 nm wavelength laser as the light source. Measurements were done at room temperature by preparing films with a thickness of 6 *µ*m at room temperature in the horizontal and vertical directions.

### Examples of the Syntheses of Diamine Compounds

### 1) Synthesis of 2,6-bis(trifluoromethyl)benzidine

1-Bromo-4-dinitro-2,6-bistrifluoromethylbenzene 3.48 g (10.3 mmol) and 4-aminophenylboronic acid hydrochloride 3.47 g (20.0 mmol), potassium carbonate (K2CO3) 9.6 g, tetrakistriphenylphosphine palladium 0.6 g (0.519 mmol) were dissolved in the mixture of toluene(20 ml), water(20 ml) and ethanol(10 ml), and the reaction solution was stirred for 20. 5 hour at 120 °C. After completion of the reaction, the solution was diluted with ethylacetate (300 mL) and washed with distilled water several times to remove salts. Anhydrous magnesium sulfate was then added to ethylacetate solution to remove water and the solvent was evaporated, followed by filtering resulting reactant being passed through a silica column to give dintro compound. (2.63 g, 7.51 mmol; yield 75.1%)

Melting point: 106 - 107 °C.

¹H NMR (DMSO-d6, 400MHz, ppm): 8.68(s, 2H), 6.84(d, J=8.2Hz, 2H), 6.56(d, J=8.5Hz, 2H), 5.35(s, NH2).

¹³C NMR (DMSO-d6, 100MHz, ppm): 149.39, 147.64, 146.36, 132.11 (q, J=30.2Hz), 129.70, 124.63(q, J=5.8Hz), 122.25(q, J=275.2Hz), 118.29, 112.30.

FTIR (KBr, cm-1): 3499, 3401 (NH2); 1620 (aromatic C=C); 1533, 1359, 1333 (NO2); 1126-1295 (C-F).

EA: Anal. Calcd for C14H8F6N2O2: C, 48.01; H, 2.30; N, 8.00.
Found: C, 49.05; H, 2.56; N, 7.71.

The said dinitro compound 5.01 g (14.3 mmol) and 10% palladium on carbon 2.5 g were poured onto a mixture of 30 mL of ethyl acetate and 30 mL of ethanol, and stirred under hydrogen gas for 20.5 hr. After the reaction, palladium carbon was removed using a filter, followed by the evaporation of ethanol and ethyl acetate to give a yellow diamine monomer. The monomer was passed through silica column and the resulting product was then vacuum sublimation to get white crystals of 2,6-bis(trifluoromethyl)benzidine. (4.53 g, 14.1 mmol; yield 99 %)

Melting point: 127-128 °C.

1H NMR (DMSO-d6, 400MHz, ppm): 7.17(s, 2H), 6.77(d, J=8.0Hz, 2H), 6.50(d, J=8.0Hz, 2H), 5.95(s, NH2), 5.08(s, NH2).

13C NMR (DMSO-d6, 100MHz, ppm): 148.19, 147.92, 131.13, 131.02 (q, J=27.5Hz), 126.00, 123.70(q, J=275.0Hz), 121.35, 113.23(q, J=6.2Hz), 112.40.

FTIR (KBr, cm-1): 3486, 3335, 3221 (NH2); 1640, 1475 (aromatic C=C); 1119-1279 (C-F).

EA: Anal. Calcd for C14H10F6N2: C, 52.51; H, 3.15; N, 8.75. Found: C, 53.71; H, 3.04; N, 8.74.

### 2) Synthesis of 2,6-bis(trifluoroemthyl)-4,4'-diaminodiphenyl ether

Disodium phosphate 18.2 g (123 mmol) and tetrabutylammonium hydrogen sulfate 2.1 g (6.20 mmol) were dissolved in a 500 mL solution of acetone and dichloromethane, followed by the addition of 4-bromo-3,5-bis-trifluoromethyl aniline 10.0 g (32.5 mmol) dropwise with a oxone and the reaction solution was stirred for 1 hour at 0° C. Potassium hydroxide was added to maintain the acidity of the reaction solution between 7.5 and 8.5. After completion of the reaction, the solution was diluted with dichloromethane and washed with distilled water several times to remove salts. Magnesium sulfate was then added to the dichloromethane solution and the solvent was filtered then evaporated and the resulting reactant was passed through a silica column to give a light yellow compound, 1-bromo-4-nitro-2,6-bis(trifluoromethyl)benzene (8.05 g, 23.8 mmol 73.3% yield).

Melting point: 56-57 °C.

1H NMR (CDC13, 400 MHz, ppm): 8.71 (s, 2H).

13C NMR (DMSO-d6, 100 MHz, ppm): 146.62, 132.48 (q, 1:319 Hz), 126.71 (q, 1:5.7 Hz), 125.63, 121.68(q, 1:2729 Hz).

The said 1-bromo-4-nitro-2,6-bis-trifluoromethyl benzene, 6.99 g (20.7 mmol) and 4-nitrophenol 3.16 g (22.7 mmol) were dissolved in 40 mL of dimethyl sulfoxide, then potassium carbonate (K2CO3) 4.29 g (31.0 mmol) was added and the mixture was stirred for 1.5 hours. The reaction was diluted with 300 mL of ethyl acetate, followed by extraction with distilled water several times to remove the dimethyl sulfoxide and salts. To the ethyl acetate solution was added anhydrous magnesium sulfate to remove water, followed by passing the resulting reactant through a silica column to obtain a yellow dinitro compound, 2,6-bis(trifluoromethyl)-4,4'-dinitrodiphenyl ether (8.20 g, 20.7 mmol; yield 100%).

1H NMR (CDC13, 400 MHz, ppm): 8.831 (s, 2H), 8.181 (d, 1:9.6 Hz, 2H), 6.891 (d, 1:9.6 Hz, 2H).

13C NMR (CDC13, 100 MHz, ppm): 162.48, 153.74, 144.91, 143.74, 128.17 (q, 1:343 Hz), 127.39 (q, 1:5.0 Hz), 125.87, 121.02 (q, 1:2748 Hz), 115.95.

The dinitro compound 8 g (20.2 mmol) and 5% palladium on carbon 4 g were poured onto a mixture of 160 mL of ethyl acetate and 160 mL of ethanol, and stirred under hydrogen gas for three days. After the reaction, palladium carbon was removed using a filter, followed by the evaporation of ethanol and ethyl acetate to yield a yellow diamine compound. The compound was passed through silica column and the resulting product was then recrystallized in a mixture of chloroform and hexane, followed by sublimation at 130° C by vacuum sublimation to get white crystals of 2,6-bis(trifluoromethyl)-4,4'-diaminodiphenyl ether (6.6 g, 19.6 mmol; yield: 97%).

Melting point: 138-139° C.

1H NMR (DMSO-d6, 400 MHz, ppm): 7.164 (s, 2H), 6.414 (m, 4H), 5.927 (s, 2H), 4.675 (s, 2H).

13C NMR (DMSO-d6, 100 MHz, ppm): 151.38, 146.61, 143.32, 138.15, 125.31 (q, 1:307 Hz), 122.83 (q,1:273.7 Hz), 115.06, 115.00, 114.56.

FTIR (KBr, cm-1):

### Preparation of dicarboxylic acid derivatives

### 2,2'-(2,6-bis(trifluoromethyl)-[1,1'-biphenyl]-4,4'-diyl)bis(1,3-dioxoisoindoline-5-carboxylic acid)

The said diamine compound 1.50g (4.68 mmol) and trimelitic anhydride 1.81 g(9.44 mmol) were dissolved in glacial acetic acid 20 mL and refluxed for 21.5 hours while stirring. After the complete reaction, the solvent was added to methanol to form precipitation and filtering the solvent would give dicarboxylic acid monomer. (2.74 g, 4.10 mmol; yield 88 %)

mp : 361-362 °C.

1H NMR (DMSO-d6, 400MHz, ppm): 13.80 (broad, COOH), 8.46 (dd, J = 7.8, 1.4 Hz, 1H), 8.43 (dd, J = 7.8, 1.4 Hz, 1H), 8.37 (dd, J = 1.4, 0.8 Hz, 1H), 8.37(s, 2H), 8.33 (dd, J = 1.4, 0.7 Hz, 1H), 8.16 (dd, J = 7.7, 0.7 Hz, 1H), 8.11 (dd, J = 7.7, 0.7 Hz, 1H), 7.57(s, 4H).

13C NMR (DMSO-d6, 100MHz, ppm): 166.08, 166.07, 165.76, 165.72, 165.71, 165.68, 138.24, 136.81, 136.51, 135.74, 135.47, 134.85, 134.74, 132.43, 132.29, 132.15, 132.01, 131.94, 130.61 (q, J = 29.7 Hz), 130.34, 128.35 (q, J = 5.7 Hz), 125.45, 124.10, 123.83, 123.58, 123.38, 122.78 (q, J = 274.9 Hz).

### Example 1 (uuBTFB-PAI)

The said 2,6-bis(trifluoromethyl)benzidine diamine monomer 0.30301 g (0.946 mmol), dicarboxylic acid monomer from said example 0.63257 g (0.946 mmol), and triphenylphosphite 1mL, pyridine 1 mL, calcium chloride 0.3 g were dissolved in NMP 4 mL and the solution was stirred at 100 °C for 8 hours. During the reaction, the proper amount of NMP was added to lower the viscosity of condensation solution, and no precipitation or gelation was observed. After the reaction was completed, the reactant was cooled to room temperature and the viscous solution was added to methanol to precipitate and filter, and then the precipitant was washed with excess amount of water, followed by hot methanol and water, followed by drying in a vacuum oven at 180 °C to obtain polymer. Small portion of synthesized polymer was dissolved in DMAc to make 6.7 wt% of DMAc solution which was cast onto glass plate and the solvent was removed by heating the plate at 190 °C under vacuum to obtain transparent and strong film.

1H NMR (DMSO-d6, 400MHz, 25°C, ppm): 11.22 (s, 1H), 10.81(s, 1H), 8.84 - 8.46 (m, 6H), 8.41 (s, 2H), 8.31 - 8.09 (m, 2H), 8.06 - 7.01 (m, 8H).

1H NMR (DMSO-d6, 400MHz, 100°C, ppm): 10.92 (s, 1H), 10.45 (s, 1H), 8.74 - 8.46 (m, 6H), 8.41 (s, 2H), 8.17 (m, 2H), 7.89 (d, J = 8.2 Hz, 2H), 7.66 (d, J = 7.9 Hz, 2H), 7.55 (d, J = 6.7 Hz, 2H), 7.31 (d, J = 8.4 Hz, 2H).

FTIR (film, cm-1):

3353(NH stretching); 1784, 1732(C=O stretching of imide); 1686 (C=O stretching of amide); 1475-1599 (Aromatic C=C); 1374 (C-N stretching of imide); 1189, 1135 (C-F in CF3); 725 (Imide ring deformation).

### Example 2 (suBTFB-PAI1)

2,2'-bis(trifluoromethyl)benzidine diamine monomer 0.19149 g (0.598 mmol), dicarboxylic acid monomer from said example 0.39972 g (0.598 mmol), and triphenylphosphite 1mL, pyridine 1 mL, calcium chloride 0.3 g were dissolved in NMP 6 mL and the solution was stirred at 100 °C for 8 hours. During the reaction, the proper amount of NMP was added to lower the viscosity of condensation solution, and no precipitation or gelation was observed. After the reaction was completed, the reactant was cooled to room temperature and the viscous solution was added to methanol to precipitate and filter, and then the precipitant was washed with excess amount of water, followed by hot methanol and water, followed by drying in a vacuum oven at 180 °C to obtain polymer. Small portion of synthesized polymer was dissolved in DMAc to make 2.5 wt% of DMAc solution which was cast onto glass plate and the solvent was removed by heating the plate at 190 °C under vacuum to obtain transparent and strong film.

1H NMR (DMSO-d6, 400MHz, 100°C, ppm): 10.70 (s, 2H), 8.71 - 8.49 (m, 4H), 8.41 (s, 2H), 8.35 (s, 2H), 8.17 (m, 4H), 7.66 (d, J = 8.0 Hz, 2H), 7.55 (d, J = 7.9 Hz, 2H), 7.41 (d, J = 8.8 Hz, 2H).

FTIR (film, cm-1):

3353 (NH stretching); 1784, 1732 (C=O stretching of imide); 1686 (C=O stretching of amide); 1476-1596(Aromatic C=C); 1377 (C-N stretching of imide); 1174, 1131 (C-F in CF3); 725 (Imide ring deformation).

### Example 3 (suBTFB-PAI2)

For adjusting reproducibility and molecular weight of Example 2, all the reaction condition was maintained as the same as example 2, except for concentration. 2,2'-bis(trifluoromethyl)benzidine diamine monomer 0.19149 g (0.598 mmol), dicarboxylic acid monomer from said example 0.39972 g (0.598 mmol), and triphenylphosphite 1mL, pyridine 1 mL, calcium chloride 0.3 g were dissolved in NMP 15 mL and the solution was stirred at 100 °C for 8 hours. During the reaction, the proper amount of NMP was added to lower the viscosity of condensation solution, and no precipitation or gelation was observed. After the reaction was completed, the reactant was cooled to room temperature and the viscous solution was added to methanol to precipitate and filter, and then the precipitant was washed with excess amount of water, followed by hot water and methanol, followed by drying in a vacuum oven at 180 °C to obtain polymer. Small portion of synthesized polymer was dissolved in DMAc to make 2.5 wt% of DMAc solution which was cast onto glass plate and the solvent was removed by heating the plate at 190 °C under vacuum to obtain transparent and strong film.

1H NMR (DMSO-d6, 400MHz, 25°C, ppm): 11.05 (s, 2H), 8.83 - 8.48 (m, 4H), 8.42 (s, 4H), 8.19 (s, 4H), 7.82 - 7.21 (m, 6H).

1H NMR (DMSO-d6, 400MHz, 100°C, ppm): 10.73 (s, 2H), 8.74 - 8.49 (m, 4H), 8.41 (s,2H), 8.36 (s, 2H), 8.18 (m, 4H), 7.66 (d, J = 8.3 Hz, 2H), 7.56 (d, J = 8.6 Hz, 2H), 7.42(d, J = 8.4 Hz, 2H).

FTIR (film, cm-1):

3352(NH stretching); 1784, 1732(C=O stretching of imide); 1686 (C=O stretching of amide); 1476 - 1595 (Aromatic C=C); 1377 (C-N stretching of imide); 1174, 1131 (C-F in CF3); 725 (Imide ring deformation).

### Example 4 (usBTFB-PAI)

2,6-Bis(trifluoromethyl)benzidine diamine monomer from the said example 0.19149 g (0.598 mmol), 2,2'-(2,2'-bis(trifluoromethyl)-[1,1'-biphenyl]-4,4'-diyl)bis(1,3-dioxoisoindoline-5-carboxylic acid) 0.39804 g (0.595 mmol) and triphenylphosphite 1mL, pyridine 1 mL, calcium chloride 0.3 g were dissolved in NMP 6 mL and the solution was stirred at 100 °C for 8 hours. During the reaction, the proper amount of NMP was added to lower the viscosity of condensation solution, and no precipitation or gelation was observed. After the reaction was completed, the reactant was cooled to room temperature and the viscous solution was added to methanol to precipitate and filter, and then the precipitant was washed with excess amount of water, followed by hot methanol and water, followed by drying in a vacuum oven at 180 °C to obtain polymer. Small portion of synthesized polymer was dissolved in DMAc to make 5 wt% of DMAc solution which was cast onto glass plate and the solvent was removed by heating the plate at 190 °C under vacuum to obtain transparent and strong film.

1H NMR (DMSO-d6, 400MHz, 25 °C, ppm): 11.25 (s, 1H), 10.83 (s, 1H), 8.85 - 8.41 (m, 6H), 8.31 - 8.04 (m, 4H), 7.92 (s, 4H), 7.72 (s, 2H), 7.32 (s, 2H).

1H NMR (DMSO-d6, 400MHz, 100°C, ppm): 10.96 (s, 1H), 10.48 (s, 1H), 8.76 - 8.43 (m, 6H), 8.29 - 8.05 (m, 4H), 7.92 (dd, J = 20.8, 8.6 Hz, 4H), 7.67 (d, J = 8.8 Hz, 2H), 7.32 (d, J = 8.6 Hz, 2H).

FTIR (film, cm-1):

3353 (NH stretching); 1784, 1731 (C=O stretching of imide); 1689 (C=O stretching of amide); 1476 - 1598 (Aromatic C=C); 1366 (C-N stretching of imide); 1179, 1136(C-F in CF3); 725(Imide ring deformation).

### Example 5 (DAN-PAI)

1,5-Diaminonaphthalene diamine monomer 0.09425 g(0.596 mmol), dicarboxylic acid derivative from said example 0.39821 g (0.596 mmol) and triphenylphosphite 1mL, pyridine 1 mL, calcium chloride 0.3 g were dissolved in NMP 8 mL and the solution was stirred at 100 °C for 8 hours. During the reaction, the proper amount of NMP was added to lower the viscosity of condensation solution, and no precipitation or gelation was observed. After the reaction was completed, the reactant was cooled to room temperature and the viscous solution was added to methanol to precipitate and filter, and then the precipitant was washed with excess amount of water, followed by hot methanol and water, followed by drying in a vacuum oven at 180 °C to obtain polymer. Small portion of synthesized polymer was dissolved in DMAc to make 3 wt% of DMAc solution which was cast onto glass plate and the solvent was removed by heating the plate at 190 °C under vacuum to obtain transparent and strong film.

1H NMR (DMSO-d6, 400MHz, 25 °C, ppm): 10.96 (s, 2H), 8.85 - 8.51 (m, 4H), 8.44 (s,2H), 8.23 (m, 2H), 8.06 (s, 2H), 7.69 (m, 8H).

1H NMR (DMSO-d6, 400MHz, 100 °C, ppm): 10.55 (d, J = 8.0 Hz, 2H), 8.79 - 8.52 (m,4H), 8.43 (s, 2H), 8.20 (d, J = 7.4 Hz, 1H), 8.16 (d, J = 7.6 Hz, 1H), 8.09 (d, J = 8.3 Hz, 2H), 7.77 (d, J = 7.4 Hz, 2H), 7.71 - 7.61 (m, 4H), 7.57 (d, J = 8.8 Hz, 2H).

FTIR ( 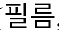 , cm-1): 3297 (NH stretching);

1783, 1731 (C=O stretching of imide); 1680 (C=O stretching of amide); 1476 -1602 (Aromatic C=C); 1377(C-N stretching of imide); 1190, 1132 (C-F in CF3); 725 (Imide ring deformation).

### Example 6 (mPDA-PAI)

m-Phenylene diamine monomer 0.06385 g (0.590 mmol), dicarboxylic acid derivative from said example 0.39466 g (0.590 mmol) and triphenylphosphite 1mL, pyridine 1 mL, calcium chloride 0.3 g were dissolved in NMP 12 mL and the solution was stirred at 100 °C for 8 hours. During the reaction, the proper amount of NMP was added to lower the viscosity of condensation solution, and no precipitation or gelation was observed. After the reaction was completed, the reactant was cooled to room temperature and the viscous solution was added to methanol to precipitate and filter, and then the precipitant was washed with excess amount of water, followed by hot methanol and water, followed by drying in a vacuum oven at 180 °C to obtain polymer. Small portion of synthesized polymer was dissolved in DMAc to make 5 wt% of DMAc solution which was cast onto glass plate and the solvent was removed by heating the plate at 190 °C under vacuum to obtain transparent and strong film.

1H NMR (DMSO-d6, 400MHz, 25 °C, ppm): 10.77 (d, J = 8.6 Hz, 2H), 8.62 (d, J = 10.9 Hz, 2H), 8.56 - 8.46 (m, 3H), 8.41 (s, 2H), 8.22 (d, J = 7.7 Hz, 1H), 8.17 (d, J = 7.9 Hz, 1H), 7.61 (s, 6H), 7.41 (s, 1H).

1H NMR (DMSO-d6, 400MHz, 100 °C, ppm): 10.51 (d, J = 6.9 Hz, 2H), 8.61 (d, J = 13.3 Hz, 2H), 8.55 (d, J = 8.3 Hz, 1H), 8.51 (d, J = 8.3 Hz, 1H), 8.40 (s, 3H), 8.18 (d, J = 7.7 Hz, 1H), 8.13 (d, J = 7.8 Hz, 1H), 7.69 - 7.49 (m, 6H), 7.39 (t, J = 8.4 Hz, 1H).

FTIR (film, cm-1):

3346 (NH stretching); 1782, 1730 (C=O stretching of imide); 1683 (C=O stretching of amide); 1476 - 1608 (Aromatic C=C); 1377 (C-N stretching of imide); 1190, 1129 (C-F in CF3); 724 (Imide ring deformation).

### Example 7 (ODA-PAI)

4,4'-Diaminodiphenyl ether diamine monomer 0.11989 g (0.599 mmol), dicarboxylic acid derivative from said example 0.40022 g (0.599 mmol) and triphenylphosphite 1mL, pyridine 1 mL, calcium chloride 0.3 g were dissolved in NMP 12 mL and the solution was stirred at 100 °C for 8 hours. During the reaction, the proper amount of NMP was added to lower the viscosity of condensation solution, and no precipitation or gelation was observed. After the reaction was completed, the reactant was cooled to room temperature and the viscous solution was added to methanol to precipitate and filter, and then the precipitant was washed with excess amount of water, followed by hot methanol and water, followed by drying in a vacuum oven at 180 °C to obtain polymer. Small portion of synthesized polymer was dissolved in DMAc to make 3.8 wt% of DMAc solution which was cast onto glass plate and the solvent was removed by heating the plate at 190 °C under vacuum to obtain transparent and strong film.

1H NMR (DMSO-d6, 400MHz, 25 °C, ppm): 10.70 (d, J = 9.3 Hz, 2H), 8.60 (d, J = 10.2 Hz, 2H), 8.55 - 8.45 (m, 2H), 8.40 (s, 2H), 8.29 - 8.11 (m, 2H), 7.86 (d, J = 8.9 Hz, 4H), 7.60 (s, 4H), 7.08 (d, J = 8.5 Hz, 4H).

1H NMR (DMSO-d6, 400MHz, 100 °C, ppm): 10.35 (d, J = 7.8 Hz, 2H), 8.58 (d, J = 14.0 Hz, 2H), 8.53 (d, J = 8.2 Hz, 1H), 8.50 (d, J = 8.2 Hz, 1H), 8.40 (s, 2H), 8.16 (d, J = 7.8 Hz, 1H), 8.11 (d, J = 7.9 Hz, 1H), 7.83 (d, J = 8.7 Hz, 4H), 7.64 (d, J = 8.2 Hz, 2H), 7.55 (d, J = 8.5 Hz, 2H), 7.08 (d, J = 8.0 Hz, 4H).

FTIR (film, cm-1):

3351(NH stretching); 1782, 1729(C=O stretching of imide); 1676 (C=O stretching of amide); 1476 - 1603 (Aromatic C=C); 1376 (C-N stretching of imide); 1222 (C-O-C); 1190, 1130(C-F in CF3); 725 (Imide ring deformation).

Following Table 1 thru 4 show the properties of polyamideimide in the examples of the present invention.

Table 1 shows the viscosity and elementary analysis of polyamideimide of example 1 through 7.

**Table 1**

| | **polymer** | **viscosity** (η_{inh.} dL/g) | Formula of PAI (formula weight) | | C | H | N |
|---|---|---|---|---|---|---|---|
| **Example 1** | uuBTFB-PAI | 1.23 | (C₄₆H₂₀F₁₂N₄O₆)ₙ | Calcd | 57.99 | 2.12 | 5.88 |
| | | | (952.66)ₙ | Found | 56.85 | 1.99 | 5.74 |
| **Example 2** | suBTFB-PAI1 | 6.93 | (C₄₆H₂₀F₁₂N₄O₆)ₙ | Calcd | 57.99 | 2.12 | 5.88 |
| | | | (952.66)ₙ | Found | 56.72 | 2.06 | 5.76 |
| **Example 3** | suBTFB-PAI2 | 3.98 | (C₄₆H₂₀F₁₂N₄O₆)ₙ | Calcd | 57.99 | 2.12 | 5.88 |
| | | | (952.66)ₙ | Found | 57.27 | 2.09 | 5.76 |
| **Example 4** | usBTFB-PAI | 3.02 | (C₄₆H₂₀F₁₂N₄O₆)ₙ | Calcd | 57.99 | 2.12 | 5.88 |
| | | | (952.66)ₙ | Found | 57.03 | 2.03 | 5.79 |
| **Example 5** | DAN-PAI | 3.11 | (C₄₂H₂₀F₆N₄O₆)ₙ | Calcd | 63.80 | 2.55 | 7.09 |
| | | | (790.62)ₙ | Found | 62.52 | 2.60 | 7.15 |
| **Example 6** | mPDA-PAI | 1.60 | (C₃₈H₁₈F₆N₄O₆)ₙ | Calcd | 61.63 | 2.45 | 7.57 |
| | | | (740.56)ₙ | Found | 60.07 | 2.54 | 7.08 |
| **Example 7** | ODA-PAI | 2.52 | (C₄₄H₂₂F₆N₄O₇)ₙ | Calcd | 63.47 | 2.66 | 6.73 |
| | | | (832.66)ₙ | Found | 60.66 | 2.87 | 6.13 |

Table 2 shows glass transition temperature, temperature at the 5 wt % loss and thermal expansion coefficient and wavelength when transmittance of light starts, transmittance at 550 nm, thickness of film.

**Table 2**

| | **polymer** | **Tₐ(°C)** | **5% weignt decrease temperature** | | **CTE (ppm/°C)** | | **Cutoff wavelength (nm)** | **Transmittance at 550 nm (%)** | **Film thickness (µm)** |
|---|---|---|---|---|---|---|---|---|---|
| | | | **nitrogen current** | **air current** | **2^{nd} scan** | **3^{rd} scan** | | | |
| **Example 1** | uuBTFB-PAI | ND | 493 | 489 | 17.5 | 18.8 | 371 | 88 | ∼80 |
| **Example 2** | suBTFB-PAI 1 | ND | 476 | 468 | 4.3 | 4.6 | 371 | 88 | ∼60 |
| **Example 3** | suBTFB-PAI 2 | ND | 478 | 484 | 4.2 | 4.4 | 371 | 88 | ∼70 |
| **Example 4** | usBTFB-PAI | ND | 485 | 486 | 4.6 | 5.7 | 371 | 89 | ∼80 |
| **Example 5** | DAN-PAI | 346 | 467 | 474 | 17.7 | 16.5 | 424 | 74 | ∼60 |
| **Example 6** | mPDA-PAI | 355 | 464 | 473 | 27.3 | 27.1 | 404 | 85 | ∼70 |
| **Example 7** | ODA-PAI | 330 | 447 | 450 | 27.0 | 26.5 | 421 | 86 | ∼60 |

In case of DSC analysis, at 10 °C/min rate of increase of temperature, it's impossible to observe glass transition temperature (Tg). Tg was measured using TMA and in case of ND, it's impossible to observe Tg.

As shown in Table 3, solubility of polyamideimide from example 1 thru example 7 toward various solvents is demonstrated.

**Table 3**

| | **polymer** | **NMP** | **DMAc** | **DMF** | **DMSO** | **m-cresol** | **THF** | **EA** | **acetone** |
|---|---|---|---|---|---|---|---|---|---|
| **Example 1** | uuBTFB-PAI | ++ | ++ | ++ | ++ | ++ | ++ | +- | +- |
| **Example 2** | suBTFB-PAI1 | ++ | ++ | ++ | ++ | ++ | ++ | - | - |
| **Example 3** | suBTFB-PAI2 | ++ | ++ | ++ | ++ | ++ | ++ | - | - |
| **Example 4** | usBTFB-PAI | ++ | ++ | ++ | ++ | ++ | ++ | - | - |
| **Example 5** | DAN-PAI | ++ | +- | - | ++ | - | - | - | - |
| **Example 6** | mPDA-PAI | ++ | ++ | ++ | ++ | ++ | - | - | - |
| **Example 7** | ODA-PAI | ++ | ++ | +- | ++ | - | - | - | - |

| | | | | | | | | | |
|---|---|---|---|---|---|---|---|---|---|
| * Solubility: ++ soluble at room temperature, +- partially soluble, - insoluble. | | | | | | | | | |

NMP : N-methyl pyrrolidone, DMAc : NN-dimethyl acetamide, DMF: NN-dimethylformaldehyde, DMSO: Dimethylsulfoxide, THF : tetrahydrofuran, EA: ethyl acetate

Polyamideimide of the present invention has excellent solubility toward NMP, DMAc, and DMSO, probably due to the inhibition of several interactions by introducing asymmetric structure to polymer.

Meanwhile, the polyamideimide is insoluble to ordinary organic solvent such as ethyl acetate or acetone, however, depending on the kinds of diamine monomers, the polyamideimide of the present invention is soluble in m-cresol, THF, NMP, DMAc, DMF and DMSO.

As shown in Tables 1 through Table 3, all polyamideimides show good solubility toward organic solvent and high thermal stability. By dissolving polyamideimide in the organic solvent and cast gives strong and transparent film with low thermal expansion coefficient.

Especially, polyamideimide of examples 1 through 4 meet the criteria such as thermal stability and optical properties applicable for plastic substrate in flexible display. Also considering the fact that the thermal expansion coefficient of pyrex glass is 4 ppm/°C, polymers of examples 2 through 4 has big advantage of no changes in the previous TFT process condition to replace glass.

Table 4 shows refractive index of polyamideimide from examples 1 through 7.

**Table 4**

| | polymer | λ (nm) | *n*_{TE}^{a} | *n*_{TM}^{b} | *n*ₐᵥ^{c} | Δ*ₙ^{d}* | ε^{e} | d(µm) |
|---|---|---|---|---|---|---|---|---|
| **Example 1** | uuBTFB-PAI | 633 | 1.625 | 1.533 | 1.594 | 0.092 | 2.79 | 5.69 |
| | | 1310 | 1.593 | 1.511 | 1.566 | 0.082 | 2.70 | 5.86 |
| **Example 2** | suBTFB-PAI1 | 633 | 1.646 | 1.499 | 1.597 | 0.147 | 2.81 | 5.54 |
| | | 1310 | 1.612 | 1.485 | 1.570 | 0.127 | 2.71 | 5.49 |
| **Example 3** | suBTFB-PAI2 | 633 | 1.645 | 1.506 | 1.599 | 0.139 | 2.81 | 13.69 |
| | | 1310 | 1.612 | 1.489 | 1.571 | 0.123 | 2.71 | 13.64 |
| **Example 4** | usBTFB-PAI | 633 | 1.641 | 1.516 | 1.599 | 0.125 | 2.81 | 3.0 |
| | | 1310 | 1.608 | 1.497 | 1.571 | 0.111 | 2.71 | 3.1 |
| **Example 5** | DAN-PAI | 633 | 1.687 | 1.548 | 1.641 | 0.139 | 2.96 | 5.43 |
| | | 1310 | 1.650 | 1.526 | 1.609 | 0.124 | 2.85 | 5.44 |
| **Example 6** | mPDA-PAI | 633 | 1.669 | 1.553 | 1.630 | 0.116 | 2.92 | 6.29 |
| | | 1310 | 1.634 | 1.532 | 1.600 | 0.102 | 2.82 | 6.54 |
| **Example 7** | ODA-PAI | 633 | 1.674 | 1.560 | 1.636 | 0.114 | 2.94 | 6.29 |
| | | 1310 | 1.638 | 1.540 | 1.605 | 0.098 | 2.83 | 6.25 |

In Table 4, a is refractive index in parallel direction, b is refractive index in perpendicular direction, c is average refractive index, d is birefringence, e is dielectric constant calculated on the basis of average refractive index (ε = 1.10 nₐᵥ²), f is thickness of film.

As shown in Table 4, despite of the rigid plane structure of polyamideimide, in all case, polyamideimide has low refractive index and low dielectric constant. This is due to the inhibition of interaction between chains of polymer by bulky trifluoromethyl group, as well as low polarizability of fluorine in polymer, and therefore, the polyamideimide of the present invention can be used as electrical and electronic optical material.

### Industrial Applicability

The present invention provides with novel polyamideimide consisting of amide and imide bond within the main chain of polymer, applicable for plastic substrate in flexible display.

## Claims

1. An asymmetric dicarboxylic acid derivative, alkali metal salts or its alkaline earth metal salts thereof, represented by Formula A, having two substituents R and R' attached to only cyclic group A of both cyclic group A and B of Formula A : 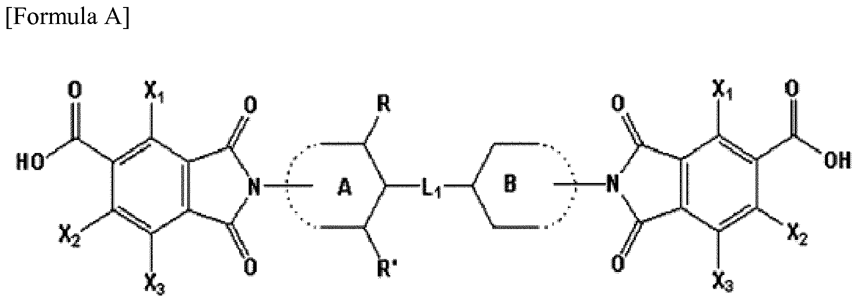 wherein R, R', X1, X2 and X3 are identical to or different from each other, and are independently selected from the group consisting of C1 to C12 alkyl group unsubstituted or substituted with one or more halogen; C1 to C12 alkoxy group unsubstituted or substituted with one or more halogen; C2 to C12 alkenyl group unsubstituted or substituted with one or more halogen; C2 to C12 alkynyl group unsubstituted or substituted with one or more halogen; C4 to C30 cycloalkyl group unsubstituted or substituted with one or more halogen; C4 to C30 cycloalkenyl group unsubstituted or substituted with one or more halogen; C6 to C30 aryl group unsubstituted or substituted with one or more halogen, C1 to C12 alkyl group, C1 to C12 alkoxy group, C1 to C12 halogenated alkyl group and/or C1 to C12 halogenated alkoxy group; C3 to C30 heteroaryl group unsubstituted or substituted with one or more halogen, C1 to C12 alkyl group, C1 to C12 alkoxy group, C1 to C12 halogenated alkyl group and/or C1 to C12 halogenated alkoxy group; and C6 to C30 arylalkyl group unsubstituted or substituted with one or more halogen, C1 to C12 alkyl group, C1 to C12 alkoxy group, C1 to C12 halogenated alkyl group and/or C1 to C12 halogenated alkoxy group;
L1 represents a direct bond, -O-, -S-, -C(=O)O-, -OC(=O)-, -C(=O)-, -SO2-, -C(R1)(R2)-, - NR3, where R1, R2 and R3 are identical to or different from each other, and are independently selected from the group consisting of hydrogen; C1 to C12 alkyl group unsubstituted or substituted with one or more halogen; C4 to C20 cycloalkyl group unsubstituted or substituted with one or more halogen; C6 to C20 aryl group unsubstituted or substituted with one or more halogen; and C3 to C20 heteroaryl group unsubstituted or substituted with one or more halogen;
said cyclic group 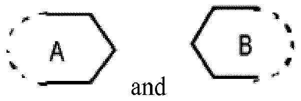 each independently represent C5 to C30 arylene or cycloalkylene unsubstituted or substituted with 5-membered or 6-membered ring which is unsubstituted or substituted or more halogen; or C5 to C30 heteroarylene or heterocycloalkylene unsubstituted or substituted with 5-membered or 6-membered ring which is unsubstituted or substituted or more halogen.

2. The asymmetric dicarboxylic acid derivatives, alkali metal salts or its alkaline earth metal salts thereof according to claim 1, wherein R and R' are identical to or different from each other, and are hydrocarbyl group comprising fluorine.

3. The asymmetric dicarboxylic acid derivatives, alkali metal salts or its alkaline earth metal salts thereof according to claim 1, wherein L is anyone selected from the group consisting of a direct bond, -O-, and -S-.

4. The asymmetric dicarboxylic acid derivatives, alkali metal salts or its alkaline earth metal salts thereof according to claim 3, wherein L is a direct bond, R and R' represent C1 to C5 alkyl substituted with fluorine, X1 to X3 are identical to or different from each other, and are selected from the group consisting of hydrogen; halogen; trifluromethyl,
pentafluoroethyl, C 1 to C5 alkyl, said cyclic group 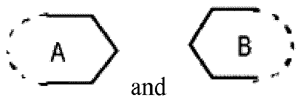 represent C6 to C12 arylene unsubstituted or substituted with one or more halogen.

5. The asymmetric dicarboxylic acid derivatives, alkali metal salts or its alkaline earth metal salts thereof according to claim 4, represented as formula A-1, wherein R and R' are each CF3 or C2F5.

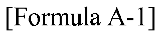

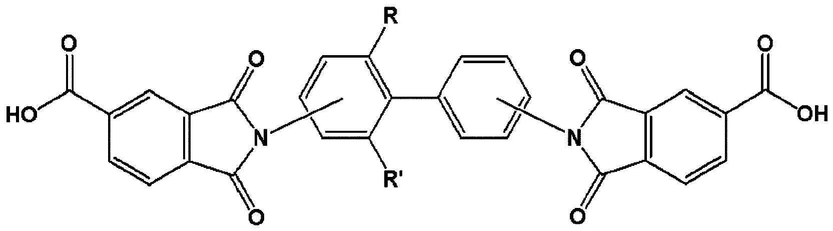

6. A process for producing an asymmetrical dicarboxylic acid derivative represented as [formula A] in Reaction 1, comprising reaction between diamine compound represented as formula A-2 and compound represented as formula A-3 in imidization reaction. 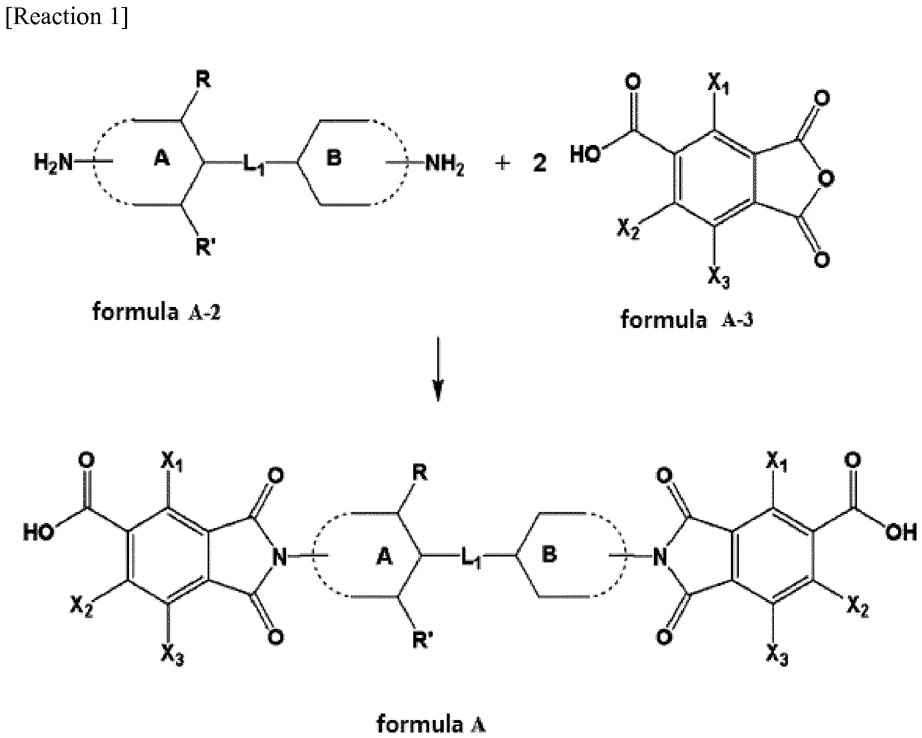 Wherein R, R', X1 to X3, and L1 and cyclic group 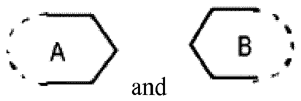 B are the same as defined in claim 1.

7. The process for producing an asymmetrical dicarboxylic acid derivative according to claim 6, wherein imidization of said Reaction 1 is dehydration reaction by glacial acetic acid.

8. Polyamideimide represented as [Formula B] or [Formula C] 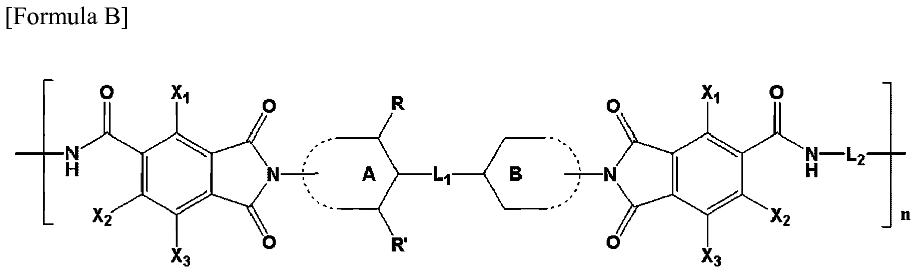 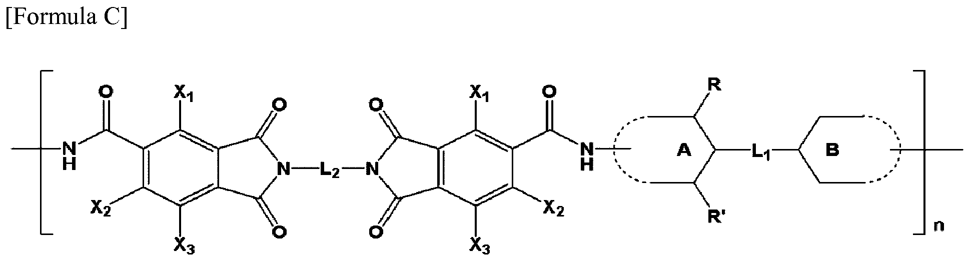 Wherein R, R', X1 to X3, and L1 and cyclic group 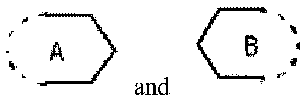 of [Formula B] and [Formula C] are the same as defined claim 1,
L2 is selected from the group consisting of C1 to C20 alkylene group unsubstituted or substituted with one or more halogen; C6 to C20 arylene group unsubstituted or substituted with one or more halogen; C6 to C30 arylene group unsubstituted or substituted with C1 to C12 alkyl group, C1 to C12 alkoxy group, C1 to C12 halogenated alkyl group, C1 to C12 halogenated alkoxy group; C2 to C20 heteroarylene group unsubstituted or substituted with one or more halogen; C4 to C20 cycloalkylene group unsubstituted or substituted with one or more halogen; and n is an integer selected from 10 to 5,000,000.

9. Polyamideimide according to claim 8, wherein R and R' are identical to or different from each other, and are hydrocarbyl group comprising fluorine.

10. Polyamideimide according to claim 8, wherein L1 is anyone selected from the group consisting of a direct bond, -O-, and -S-.

11. Polyamideimide according to claim 10, wherein L1 is direct bond; L2 is selected from a group consisting of C1 to C12 alkylene, C6 to C12 arylene, C6 to C16 arylene substituted with C1 to C12 halogenated alkyl, C2 to C12 heteroarylene; R and R' may independently represent C1 to C5 alkyl substituted with fluorine, X1 through X3 are identical to or different from each other, and are independently selected from the group consisting of hydrogen, halogen, trifluromethyl, C1 to C5 alkyl, C1 to C5 alkoxy; said cyclic group 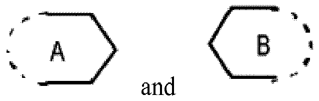 each represent C6 to C12 arylene unsubstituted or substituted with one or more halogen.

12. Polyamideimide according to claim 11, wherein L2 may be selected from a group consisting of

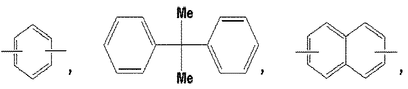

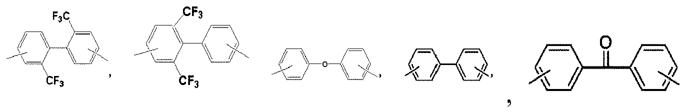

13. Polyamideimide according to claim 12, represented as Formula B-1 or Formula C-1. 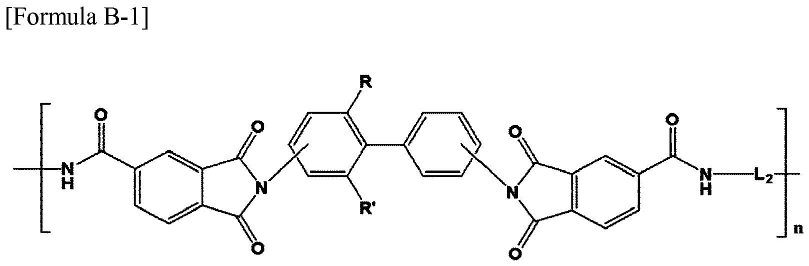 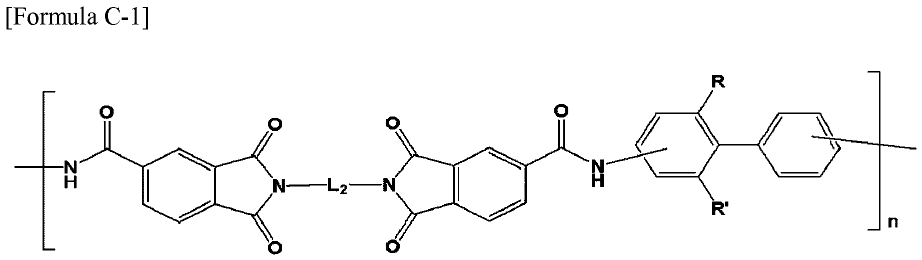 Wherein said R and R' each independently represent CF3 or C2F5;
L2 may be selected from a group consisting of

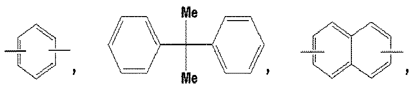

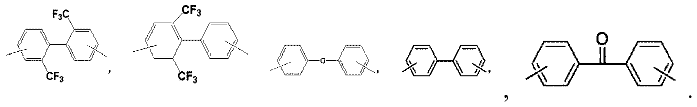

14. A process for producing the polyamideimide represented as formula B via amidation condensation between asymmetrical dicarboxylic acid derivative represented as formula A and diamine monomer represented as formula B-2 in Reaction 2; wherein R, R', X1 to X3,
and L1 and cyclic group 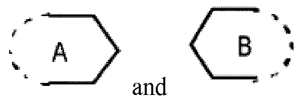 are the same as defined in claim 8.

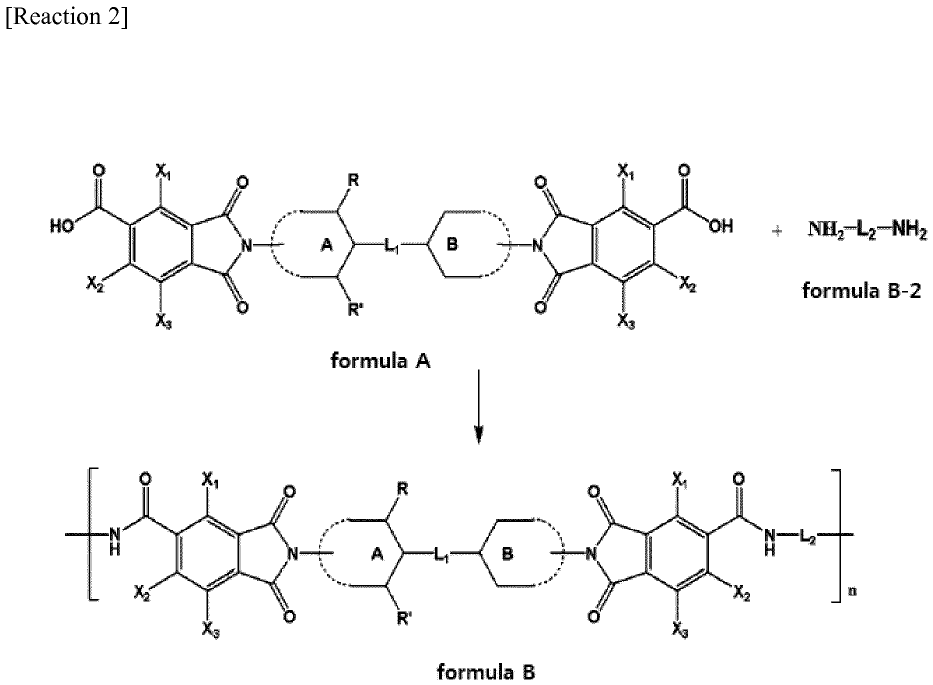

15. A process for producing the polyamideimide represented as formula C comprising steps of a) imidization condensation between trimelitic anhydride represented as formula A-3 and diamine monomer represented as formula B-2 to give dicarboxylic acid derivatives of formula C-3, in Reaction 3 and b) amidation condensation between dicarboxylic acid derivative of formula C-3 with diamine compound of formula A-2 in Reaction 4; wherein
R, R', X1 to X3, and L1, L2, n and cyclic group 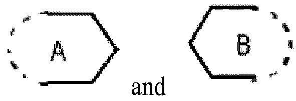 of Reaction 3 and Reaction 4 are the same as defined in claim 8.

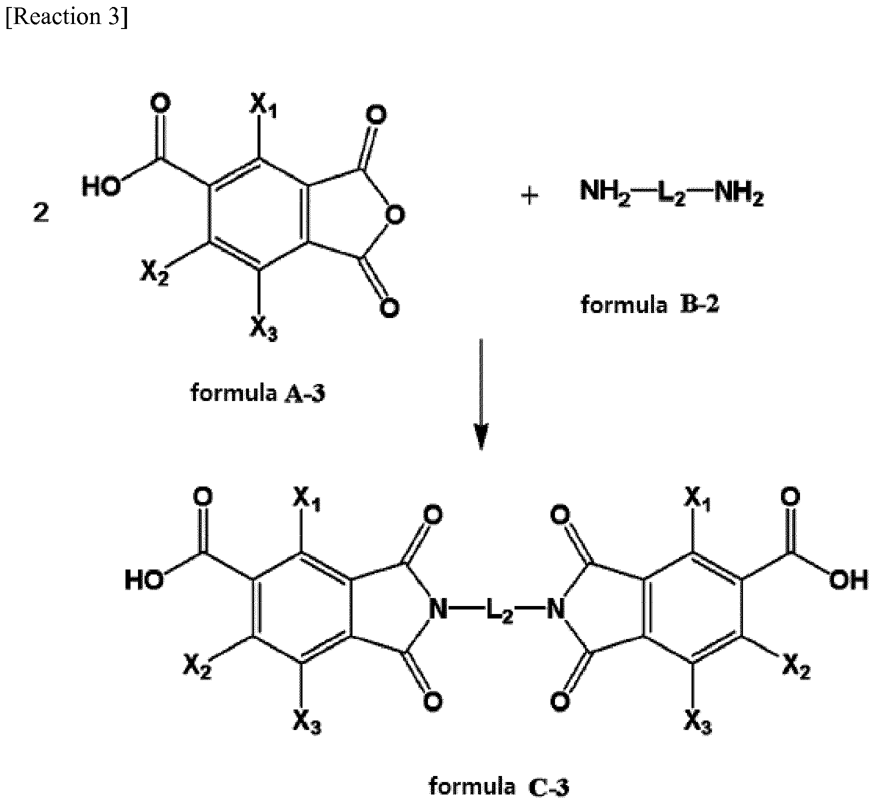

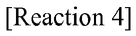

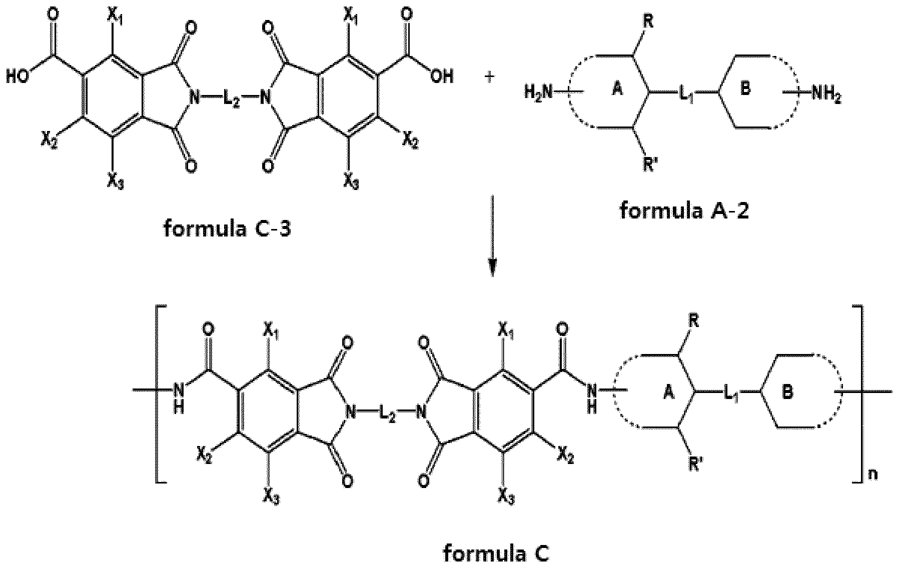

16. A process for producing the polyamideimide according to either claim 14 or claim 15, wherein diamine compound represented as formula B-2 is any one selected from the group consisting of 2,2'-bis(trifluoromethyl)benzidine, 2,6-bis(trifluoromethyl)benzidine, p-phenylenediamine, m-phenylenediamine, p-aminobenzylamine, m-aminobenzylamine, 4,4'-diaminodiphenylmethane, 3,4'-diaminodiphenylmethane, 3,3'-diaminodiphenylmethane, 4,4'-diaminodiphenylethane, 4,4'-diaminobenzanilide, 4,4'-diaminodiphenyl ether, 3,4'-diaminodiphenyl ether, 3,3'-diaminodiphenyl ether, 2,4'-diaminodiphenyl ether, 2,2'-diaminodiphenyl ether, 2,3'-diaminodiphenyl ether, 1,4-bis(4-aminophenoxy)benzene, 1,4-bis(3-aminophenoxy)benzene, 1,3-bis(4-aminophenoxy)benzene, 1,3-bis(3-aminophenoxy)benzene, 2,2-bis[4-(4-aminophenoxy)phenyl)]propane, 2,2-bis[4-(3-aminophenoxy)phenyl)]propane, bis(4-aminophenyl)sulfide, bis(3-aminophenyl)sulfide, 3,4-diaminophenyl sulfide, bis(4-aminophenyl)sulfoxide, bis(3-aminophenyl)sulfoxide, 3,4-diaminophenyl sulfoxide, bis(4-aminophenyl)sulfone, bis(3-aminophenyl)sulfone, 3,4-diaminophenyl sulfone, 4,4'-diaminobenzophenone, 3,4'-diaminobenzophenone, 3,3'-diaminobenzophenone, bis[4-(4-aminophenoxy)phenyl]sulfone, bis[4-(3-aminophenoxy)phenyl]sulfone, bis[4-(4-aminophenoxy)phenyl] ether, 1,4-bis[4-(3-aminophenoxy)benzoyl]benzene, 1,3-bis[4-(3-aminophenoxy)benzoyl]benzene, 4,4'-bis[3-(4-aminophenoxy)benzoyl]diphenyl ether, 4,4'-bis[3-(3-aminophenoxy)benzoyl]diphenyl ether, 4,4'-bis[4-(4-amino-α,α-dimethylbenzyl)phenoxy]benzophenone, 4,4'-bis[4-(4-amino-α,α-dimethylbenzyl)phenoxy]diphenyl sulfone, bis[4-{4-(4-aminophenoxy)phenoxy}phenyl]sulfone, 1,4-bis[4-(4-aminophenoxy)-α,α-dimethylbenzyl]benzene, 1,3-bis[4-(4-aminophenoxy)-α,α-dimethylbenzyl]benzene, 2,2-bis[4-(4-aminophenoxy)phenyl]-1,1,1,3,3,3-hexafluoropropane, 2,2-bis[4-(3-aminophenoxy)phenyl]-1,1,1,3,3,3-hexafluoropropane, 1,3-diaminonaphthalene, 1,4-diamino naphthalene, 1,5-diaminonaphthalene, 2,6-diaminonaphthalene, 2,2'-dimethyl-4,4'-diaminobiphenyl, 3,3'-dimethyl-4,4'-diaminobiphenyl, 2,2'-ditrifluoromethyl-4,4'-diaminobiphenyl, 3,3'-ditrifluoromethyl-4,4'-diaminobiphenyl, 5-amino-1-(4'-aminophenyl)-1,3,3-trimethylindane,
6-amino-1-(4'-aminophenyl)-1,3,3-trimethylindane, 2,2-bis[4-(4-aminophenoxy)phenyl)]propane, 2,2-bis[4-(4-aminophenoxy)phenyl)]hexafluoropropane, 2,2-bis(4-aminophenyl)hexafluoropropane, 2,2-bis[4-(4-aminophenoxy)phenyl]sulfone, 1,4-bis(4-aminophenoxy)benzene, 1,3-bis(4-aminophenoxy)benzene, 1,3-bis(3-aminophenoxy)benzene, 9,9-bis(4-aminophenyl)-10-hydroanthracene, 2,7-diaminofluorene, 9,9-dimethyl-2,7-diaminofluorene, 9,9-bis(4-aminophenyl)fluorene, 4,4'-methylene bis(2-chloroaniline), 2,2',5,5'-tetrachloro-4,4'-diaminobiphenyl, 2,2'-dichloro-4,4'-diamino-5,5'-dimethoxybiphenyl, 3,3'-dimethoxy-4,4'-diaminobiphenyl, 4,4'-(p-phenyleneisopropylidene)bisaniline, 4,4'-(m-phenyleneisopropylidene)bisaniline, 2,2'-bis[4-(4-amino-2-trifluoromethylphenoxy) phenyl]hexafluoropropane, 4,4'-diamino-2,2'-bis(trifluoromethyl)biphenyl, 4,4'-bis[(4-amino-2-trifluoromethyl)phenoxy]-octafluorobiphenyl.

17. A process for producing the polyamideimide according to claim 16, wherein diamine compound represented as formula B-2 is mixed with diamine different from diamine of formula B-2, said different diamine is any one selected from the group consisting of 2,2'-bis(trifluoromethyl)benzidine, 2,6-bis(Trifluoromethyl)benzidine, p-phenylenediamine, m-phenylenediamine, p-aminobenzylamine, m-aminobenzylamine, 4,4'-diaminodiphenylmethane, 3,4'-diaminodiphenylmethane, 3,3'-diaminodiphenylmethane, 4,4'-diaminodiphenylethane, 4,4'-diaminobenzanilide, 4,4'-diaminodiphenyl ether, 3,4'-diaminodiphenyl ether, 3,3'-diaminodiphenyl ether, 2,4'-diaminodiphenyl ether, 2,2'-diaminodiphenyl ether, 2,3'-diaminodiphenyl ether, 1,4-bis(4-aminophenoxy)benzene, 1,4-bis(3-aminophenoxy)benzene, 1,3-bis(4-aminophenoxy)benzene, 1,3-bis(3-aminophenoxy)benzene, 2,2-bis[4-(4-aminophenoxy)phenyl]propane, 2,2-bis[4-(3-aminophenoxy)phenyl]propane, bis(4-aminophenyl)sulfide, bis(3-aminophenyl)sulfide, 3,4-diaminophenyl sulfide, bis(4-aminophenyl)sulfoxide, bis(3-aminophenyl)sulfoxide, 3,4-diaminophenyl sulfoxide, bis(4-aminophenyl)sulfone, bis(3-aminophenyl)sulfone, 3,4-diaminophenyl sulfone, 4,4'-diaminobenzophenone, 3,4'-diaminobenzophenone, 3,3'-diaminobenzophenone, bis[4-(4-aminophenoxy)phenyl]sulfone, bis[4-(3-aminophenoxy)phenyl]sulfone, bis[4-(4-aminophenoxy)phenyl]ether, 1,4-bis[4-(3-aminophenoxy)benzoyl]benzene, 1,3-bis[4-(3-aminophenoxy)benzoyl]benzene, 4,4'-bis[3-(4-aminophenoxy)benzoyl]diphenyl ether, 4,4'-bis[3-(3-aminophenoxy)benzoyl]diphenyl ether, 4,4'-bis[4-(4-amino-α,α-dimethylbenzyl)phenoxy]benzophenone, 4,4'-bis[4-(4-amino-α,α-dimethylbenzyl)phenoxy]diphenyl sulfone, bis[4-{4-(4-aminophenoxy)phenoxy}phenyl]sulfone, 1,4-bis[4-(4-aminophenoxy)-α,α-dimethylbenzyl]benzene, 1,3-bis[4-(4-aminophenoxy)-α,α-dimethylbenzyl]benzene, 2,2-bis[4-(4-aminophenoxy)phenyl]-1,1,1,3,3,3-hexafluoropropane, 2,2-bis[4-(3-aminophenoxy)phenyl]-1,1,1,3,3,3-hexafluoropropane, 1,3-diaminonaphthalene, 1,4-diaminonaphthalene, 1,5-diaminonaphthalene, 2,6-diaminonaphthalene, 2,2'-dimethyl-4,4'-diaminobiphenyl, 3,3'-dimethyl-4,4'-diaminobiphenyl, 2,2'-ditrifluoromethyl-4,4'-diaminobiphenyl, 3,3'-ditrifluoromethyl-4,4'-diaminobiphenyl, 5-amino-1-(4'-aminophenyl)-1,3,3-trimethylindane, 6-amino-1-(4'-aminophenyl)-1,3,3-trimethylindane, 2,2-bis[4-(4-aminophenoxy)phenyl]propane, 2,2-bis[4-(4-aminophenoxy)phenyl]hexafluoropropane, 2,2-bis(4-aminophenyl)hexafluoropropane, 2,2-bis[4-(4-aminophenoxy)phenyl]sulfone, 1,4-bis(4-aminophenoxy)benzene, 1,3-bis(4-aminophenoxy)benzene, 1,3-bis(3-aminophenoxy)benzene, 9,9-bis(4-aminophenyl)-10-hydroanthracene, 2,7-diaminofluorene, 9,9-dimethyl-2,7-diaminofluorene, 9,9-bis(4-aminophenyl)fluorene, 4,4'-methylene-bis(2-chloroaniline), 2,2',5,5'-tetrachloro-4,4'-diaminobiphenyl, 2,2'-dichloro-4,4'-diamino-5,5'-dimethoxybiphenyl, 3,3'-dimethoxy-4,4'-diaminobiphenyl, 4,4'-(p-phenyleneisopropylidene)bisaniline, 4,4'-(m-phenyleneisopropylidene)bisaniline, 2,2'-bis[4-(4-amino-2-trifluoromethylphenoxy)phenyl]hexafluoropropane, 4,4'-diamino-2,2'-Bis(trifluoromethyl)biphenyl, 4,4'-bis[(4-amino-2-trifluoromethyl)phenoxy]-octafluorobiphenyl, 1,1-methaxylylenediamine, 1,3-propanediamine, tetramethylenediamine, pentamethylenediamine, hexamethylenediamine, heptamethylenediamine, octamethylenediamine, nonamethylenediamine, 1,4-diaminocyclohexane, isophoronediamine, tetrahydrodicyclopentadienylenediamine, hexahydro-4,7-methanoindanylenedimethylenediamine, tricyclo[6,2,1,02.7]-undecyclenedimethyldiamine, 4,4'-methylenebis(cyclohexylamine), 2,3-diaminopyridine, 2,6-diaminopyridine, 3,4-diaminopyridine, 2,4-diaminopyrimidine, 5,6-diamino-2,3-dicyanopyrazine, 5,6-diamino-2,4-dihydroxypyrimidine, 2,4-diamino-6-dimethylamino-1,3,5-triazine, 1,4-bis(3-aminopropyl)piperazine, 2,4-diamino-6-isopropoxy-1,3,5-triazine, 2,4-diamino-6-methoxy-1,3,5-triazine, 2,4-diamino-6-phenyl-1,3,5-triazine, 2,4-diamino-6-methyl-s-triazine, 2,4-diamino-1,3,5-triazine, 4,6-diamino-2-vinyl-s-triazine, 2,4-diamino-5-phenylthiazole, 2,6-diaminopurine, 5,6-diamino-1,3-dimethyluracil, 3,5-diamino-1,2,4-triazole, 6,9-diamino-2-ethoxyacridine lactate, 3,8-diamino-6-phenylphenanthridine, 1,4-diaminopiperazine, 3,6-diaminoacridine, bis(4-aminophenyl)phenylamine, 3,6-diaminocarbazole, N-methyl-3,6-diaminocarbazole, N-ethyl-3,6-diaminocarbazole, N-phenyl-3,6-diaminocarbazole, N,N'-di(4-aminophenyl)-benzidine.

18. A process for producing the polyamideimide according to either claim 14 or claim 15, wherein amidation condensation involves with dissolving diamine monomer and dicarboxylic acid derivative monomer in organic solvent to stir at 80 to 200 °C to complete amidation reaction.

19. A process for producing the polyamideimide according claim 18, wherein the organic solvent may be selected from a group consisting of N,N-dimethylformamide (DMF), N,N-dimethyl acetamide (DMAc), N-methyl pyrrolidone (NMP), and dimethyl sulfoxide (DMSO).

20. A film which is prepared by dissolving polyamideimide according to any claim of claims 8 through 13 in polar aprotic organic solvent or phenolic solvent, followed by drying the said solvent.

21. A film according to claim 20, wherein the said polar aprotic organic solvent may be selected from a group consisting of N,N-dimethylformamide (DMF), N,N-dimethyl acetamide (DMAc), N-methyl pyrrolidone (NMP), dimethyl sulfoxide (DMSO), and tetrahydrofuran (THF), and phenolic solvent may be selected from a group consisting of phenol, o-cresol, m-cresol, and p-cresol.

22. Diamine represented as [Formula D] wherein two substituents R and R' are substituted on one side of cyclic A asymmetrically. 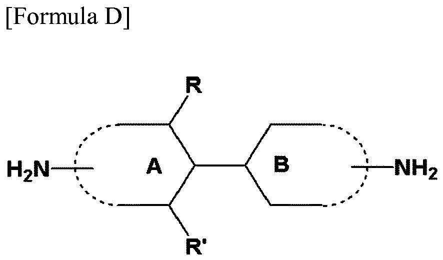 Wherein R, R' and cyclic group 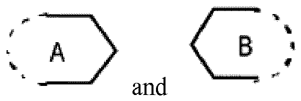 are the same as defined in claim 1.

23. Diamine according to 22, wherein R and R' are identical to or different from each other, and are hydrocarbyl group comprising fluorine.

24. Diamine according to 23,
Wherein R and R' are C1 to C5 alkyl comprising fluorine, cyclic group 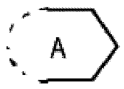 and 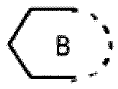 represent C6 to C12 arylene unsubstituted or substituted with one or more halogen.

25. Diamine according to 24 represented as formula D-1. 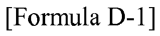 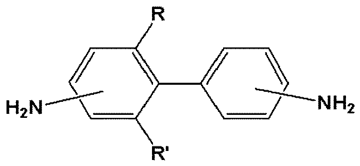 Wherein said R and R' each independently represent CF3 or C2F5;

26. A process for producing a diamine represented as Formula D, comprising steps of a) reacting nitro compound of formula D-2 with amine compound of formula D-3 or nitro compound of formula D-4 via Suzuki coupling to give either mononitro compound of formula D-5 or dinitro compound of formula D-6 shown in Reaction 5; and b) reducing either mononitro compound of formula D-5 or dinitro compound of formula D-6 via reaction 6, reducing nitro group to amine, wherein R, R' and cyclic group 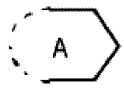 and 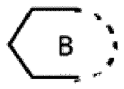 of reaction 5 and reaction 6 is the same as defined in claim 1 and X is halogen selected from I, Br, and Cl.

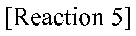

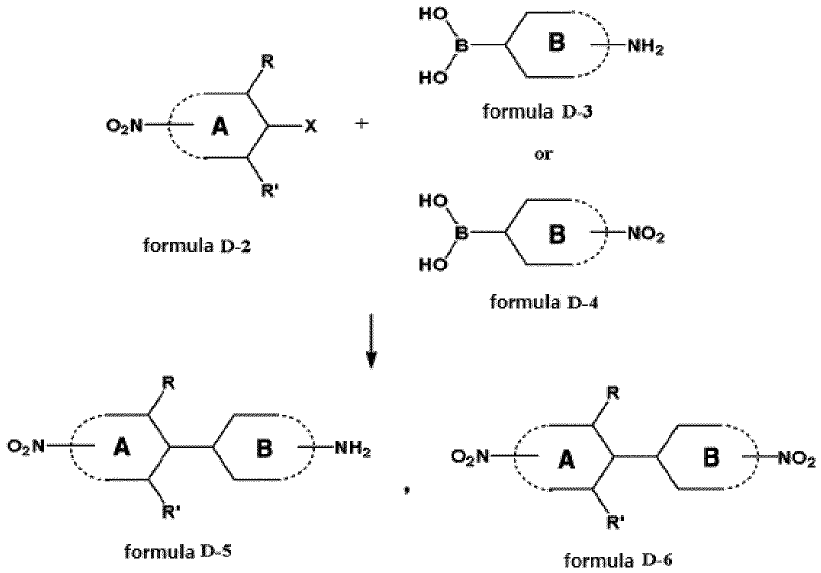

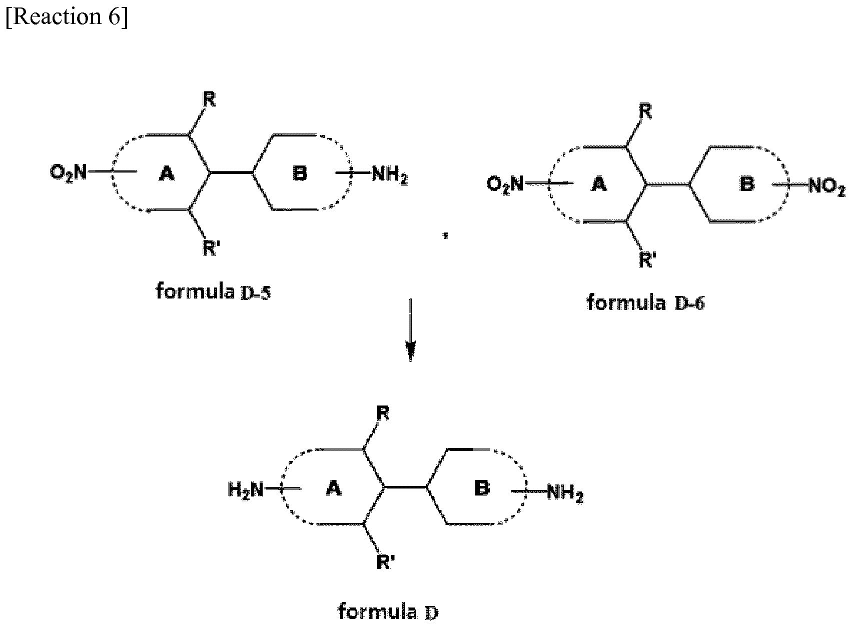

27. The process for producing diamine according to 26, wherein in step a), Suzuki coupling is done with palladium catalyst.
